# EUROPEAN PATENT APPLICATION

(11) **EP 4 678 161 A2**
(43) Date of publication of application: **14.01.2026**
(21) Application number: 25184783.6
(22) Date of filing: 15.04.2021
(51) Int. Cl.: A61K 8/66

(54) **COMPOSITIONS AND METHODS FOR TREATING HAIR FOLLICLE-LINKED CONDITIONS**

(30) Priority: 15.04.2020 EP 20169540
(62) Divisional of application: 21728435.5
(71) Applicant: Henlez ApS, 2100 København Ø (DK)
(72) Inventor: Mouritsen, Jeppe Christian, 2100 København Ø (DK); Nørregaard-Sarup, Rikke, 2100 København Ø (DK); Østergaard, Peter Rahbek, 2100 København Ø (DK)
(74) Representative: Inspicos P/S

(57) **Abstract**

Disclosed are pharmaceutical and cosmetic compositions comprising one or more glutamyl endopeptidases. The glutamyl endopeptidases are capable of catalysing the hydrolysis of keratinocyte effector molecules such as protein keratins that support the function and integrity of the human skin and its appendages, including keratins in the hair follicle, that are supported by keratinocytes to maintain the biological barrier, function and structure. The compositions are useful in treating follicular dermatoses and hair follicle-linked conditions, such as Hirsutism and Hidradenitis suppurativa. The compositions may also be used to loosen hair from the skin and thereby be used for removing undesired hair from the human body.

## Description

The present invention comprises a filing listing provided in electronical format, and the content of this listing is incorporated by reference.

### Field of Invention

The present invention relates to compositions for treating hair follicle-linked conditions. The compositions comprise at least one enzyme capable of catalysing the hydrolysis of key effector molecules, such as protein keratins or keratin-associated proteins produced by keratinocytes, during the process of tissue generation by keratinization. Keratinization allows keratinocytes to anchor skin layers together and ensures functional appendages, such as hair in its follicle. Overproduction of said keratins during pathological hyperkeratosis may lead to various hair follicle-linked skin conditions or follicular occlusion-dependent syndromes that cause follicular dermatoses. The invention involves compositions that facilitate the disruption of the hair root tissue and allows hydrolysis of hair follicle protein such as hair anchoring keratins by enzyme catalysis to facilitate removal of whole hair shafts with significantly less force than an untreated hair from the follicle of the same individual, and to facilitate the disruption of the root tissue in the hair follicle to avoid hyperkeratosis, hair shaft abnormalities, occlusion and dermatose development. The invention further relates to the use of such compositions for the treatment, intervention or prevention of hair follicle-linked conditions and follicular dermatoses such as Hirsutism, Hypertrichosis, or Pseudofolliculitis barbae; or Acne vulgaris, Keratosis pilaris, Dowling-Degos disease, Hailey-Hailey disease, Mammilary fistula; or follicular occlusion tetrad syndromes including Acne conglobata, Pilonidal sinus disease, Dissecting cellulitis of the scalp and Hidradenitis suppurativa; or for esthetic and cosmetic removal of unwanted hair from skin on the human body.

### Background

The human skin is the protective and regulatory biological barrier to the environment and is essential for homeostasis and health. It has several different appendages including nails, sweat glands and hair follicles. The structure, function and integrity of the skin are both dependent on hydrophobic lipids and keratinization protein. Keratinization is the process where a subset of protein keratins and keratin-associated proteins are paired and crosslinked by covalent and non-covalent interactions to form a structural network of filaments and resistant tissue. The stratum corneum is the outermost dead layer of skin that mainly comprises keratin-filled, interconnected, dead keratinocytes and is the main barrier to penetration of foreign molecules. It is regulated by the level of desquamation that is controlled by endogenous proteases and inhibitors. Beneath is the more active epidermis comprised of many proliferating keratinocytes that over time differentiates into stratum corneum cells and keratinize. Some stable keratinocytes constitutively express keratins such as K1, K2, K5 and K14. Others are hyperproliferative keratinocytes that in response to external stress or disease characteristically express keratins such as K17, K16 and the keratin 6 family (https://doi.org/10.5772/intechopen.79050).

In the hair follicles, hair shafts are shaped and anchored by the inner root sheath (IRS) and the outer root sheath (ORS) allowing the hair to grow from the dermal papilla. Healthy hair growth thus depends on root sheaths and their keratinocytes. The IRS is comprised of distinct sub layers of the Henle, Huxley and cuticle layers (CL), together enclosing the hair shaft. The IRS gradually keratinize by epidermal keratinization upwards from the dermal papilla to the isthmus, where it is desquamated to facilitate the exit of the bare hair shaft through the follicular canal and through the skin. Healthy hair growth is orchestrated by bidirectional cellular signals between the IRS and the ORS. While, the IRS primarily undergo epidermal keratinization, the ORS keratinize by trichilemmal keratinization from the infundibulum, just above the isthmus. Due to the central role of keratinization in barrier function and integrity, many serious dermatoses of the skin and its appendages arise from dysregulated keratinization.

It can be recognized by a person skilled in the art that follicular occlusion arises from hyperkeratosis and dysregulated keratinization of the ORS keratinocyte proteins such as the keratins. It follows that disrupting ORS tissue at any stage of differentiation by hydrolytic enzymes reaching the site of said dyskeratosis, will avoid development of the main skin pathology that is associated with the burden of said dermatoses which would otherwise occur. The primary pathophysiological site of action for topical enzymes would in most follicular dermatoses triggered by ORS dyskeratosis, be the infundibulum. The infundibulum of terminal hair follicles from which most hair follicle-linked conditions arise is on average 500-660 µm deep (https://doi.org/10.1007/s00441-014-1999-1).

One broadly applied model of human skin and hair follicle in drug and cosmetics development is, to persons skilled in the art, pig ears due to the similar physiology. An accessible in vivo model of hyperkeratosis is the mouse where distinct elements of skin conditions can be studied separately. Finally, skin biopsies from human subjects, including patients, offer the most valuable and realistic tissue model, because skin conditions and dermatoses are largely confined to the skin.

Hair follicles are normally distributed across all non-mucosal skin, but the type and appearance depends on several factors including age, gender and hormone signaling. It is for instance well known that mature male humans predominantly have coarse and pigmented hair growth on many places on the body, such as face, neck, extremities, trunk, back, buttocks, groin, where females typically have less widely distribution of these types of hair, also after puberty. Removal of undesired hair is currently performed by a large part of the global population using both physical and chemical methods. Physical devices enable hair removal in the form if shaving, laser treatment, plucking/epilation or waxing. Depilation by dissolving the hair shaft is done using strong chemicals, typically involving thioglycolates and high alkaline pH, that have the disadvantage of being highly reactive which can cause severe skin irritation in addition to not removing the full hair and its root as intended, giving a shortly lasting result. Shaving also leaves small visible hairs on the skin, routinely causes skin irritation and there is a risk of cutting the skin. Hair removal by pulling out the root using mechanical means is painful, inconvenient and rely on new hairs being of a certain length to be removed again. Lasers are applied to irreversibly damage the hair follicle to suppress hair growth, but the method requires multiple, costly treatments by a specialist, is painful, and may lead to burning and scarring. Only 50% of the treated patients respond beneficially to this method of hair removal, as it is not applicable for all hair colors, skin areas, skin colors or skintypes, because its efficacy is directly dependent upon melanin content.

Mulitple conditions and diseases are connected with dysfunctional keratinization such as excessive or pathological hair growth, hyperkeratosis and occlusion of the hair follicle and skin such as Hirsutism, Hypertrichosis or Pseudofolliculitis barbae; or skin diseases that are induced by follicular hyperkeratosis or follicular occlusion that leads to development of Acne vulgaris, Keratosis pilaris, Dowling-Degos disease, Hailey-Hailey disease, Mammilary fistula or follicular occlusion tetrad syndromes including Acne conglobata, Pilonidal sinus disease, Dissecting cellulitis of the scalp and Hidradenitis suppurativa.

Both males and females globally suffer from Hirsutism. It is estimated that approximately 5-15% of females suffer from Hirsutism to varying degrees. It is characterized by coarse and pigmented male-pattern hair growth on unusual parts of the body such as the upper lip and face, chest, back, arms, legs, groin, and buttocks. In Hirsutism patients, symptoms are caused by excess male androgen hormone signaling stimulating the hair follicles. Roughly 400.000 females in the United States of America (USA) alone have severe clinical Hirsutism. Self-reports reveal that daily hair removal is necessary for their normal well-being, hence demonstrating a large unmed medical need.

1% of the global population suffers from Hidradenitis suppurativa. It is a chronic, progressive, and recurring disease of the hair follicle associated with multiple, serious co-morbidities and very low quality of life. In Hidradenitis suppurativa and multiple related conditions, the hallmark disease-triggering event is hyperkeratosis in the hair follicle infundibulum, resulting in follicular occlusion. In Hidradenitis suppurativa, follicular occlusion leads to follicular rupture that leaks crosslinked keratin aggregates into the dermis, which induces a strong inflammatory response leading to deep-seated, purulent, and painful cystic lesions. These lesions are typically localized to the axillae, buttocks, groin, skin folds such as those seen in the overweight and under the breasts in females. Three quarters of the patients are females. Patients typically debut in puberty with a lifelong prognosis of physical and mental scarring and disability. Disease severity stages are characterized as mild (Hurley stage 1), moderate (Hurley stage 2) and severe (Hurley stage 3) with a distribution in the patient population of 68%, 28% and 4%, respectively. In the moderately to severely ill patients, the skin cysts progress to sinus tunneling and fibrotic, disfiguring and disabling skin scarring.

Only 800.000 patients or about 10% of the patient popoulation in the United States of America, Japan, Spain, Italy, France, Great Britain, and Germany are currently diagnosed with and treated for Hidradenitis suppurativa at varying levels of disease severity. Both diagnosed and undiagnosed patients have a large unmet medical need because very limited effective therapeutic solutions are available and treatment responses are highly individual. Treating sensitive and exposed skin that may unintentionally signal disease to other people is particularly challenging to deal with for the patients, and poor treatment results readily lead to further physical and psychological distress in patients. In addition, regular hair removal is very difficult, due to their sensitive skin. First-line therapy includes combinations of systemic antibiotics and hormones, and Humira^{®}, an anti-tumor necrosis factor alpha (TNFalpha) biologic monoclonal antibody drug was approved for moderate to severe disease by USA Food and Drug Administration via the orphan drug track in 2015. Humira^{®}'s high cost, limited disease controlling effect in Hidradenitis suppurava, and the risk of serious and even life-threatening adverse events and many contraindications greatly restricts its use. It is estimated in Western countries, that the current lack of useful treatment opportunities leaves 46% of patients dissatisfied with disease control, 43% report an extremely negative impact of their disease to their quality of life, and 83% of patients undergo skin surgery (https://doi.org/10.1016/j.jaad.2019.06.1301). The need for preventive therapy and options to allow better disease control is thus required. Follicular hyperkeratosis during hair growth and its occlusion is a central bottleneck event of a multitude of skin conditions. Interferring with disease at this early stage in the disease development will therefore be much more effective compared to existing therapeutics. The topical keratinolytic chemical benzene-1,3-diol that is also used for treatment of Acne vulgaris has shown unique but insufficient disease controlling benefits in a few phase II clinical trials with Hidradenitis suppurativa patients in high doses. Topical benzoyl peroxide is still under clinical trial investigation as an option to relieve Hidradenitis suppurativa.

Laser hair removal is also currently studied in clinical trials with the purpose of killing the follicular stem cells, thus both avoiding follicular hair growth and hyperkeratosis. The first results have indicated a limited preventive efficacy for Hidradenitis suppurativa. In addition, this treatment is not accessible to many patients in less developed and poor countries as it requires costly equipment, expertise and certified operators for safe use. Due to sore and inflamed lesions, particularly in commonly affected intimate areas with thin, inaccessible and sensitive skin, treatment of all affected body areas is in most cases difficult.

For intervention of unwanted hair growth, hair pathologies such as Hirsutism, and follicular dermatoses, such as Hidradenitis suppurativa and multiple related hair follicle-linked conditions as previously listed, with a therapeutic or cosmetic need of better therapies targeting the responsible keratinocytes and their effector molecules, such as the root sheath protein keratins surrounding the hair shaft, enzyme-based therapies constitute a novel interventive therapeutic drug with great promise to prevent and control progression early, safely, mildly and in a userfriendly manner.

### Short description of the invention

The invention relates to compositions comprising one or more enzymes capable of catalysing the hydrolysis of keratinocyte effector molecules such as protein keratins that support the integrity of the human skin and its appendages such as hair follicles that are supported by keratinocytes to maintain the biological barrier, function and structure, where the composition is:
a. a pharmaceutical composition, comprising at least one pharmaceutically acceptable ingredient in addition to one or more enzymes capable of catalysing the hydrolysis of keratinocyte effector molecules such as protein keratins that support the function and integrity of the human skin and its appendages such as the hair follicle; or
b. a cosmetic composition, comprising at least one cosmetically acceptable ingredient in addition to one or more enzymes capable of catalysing the hydrolysis of keratinocyte effector molecules such as protein keratins that support the function and integrity of the human skin and its appendages such as the hair follicle.

The invention also relates to a medical device comprising compositions of the invention, such as; a medical patch with for example dissolving microneedles embedded with enzyme; or a device fitted with sturdy microneedles designed to penetrate the primary, outermost skin barrier and thus enhance delivery of enzyme to the site of action. The invention further relates to the use of the inventive compositions or the medical device; for treating hair follicle-linked conditions. The use may be for medical use to treat conditions such as Hirsutism, Hypertrichosis, or Pseudofolliculitis barbae; or skin diseases that are induced by follicular hyperkeratosis or other conditions of excessive or pathological hair growth triggered by keratinization abnormalities in the follicular hair sheaths including ORS, IRS and shaft such as Pseudofolliculitis barbae, or skin diseases induced by follicular hyperkeratosis and follicular occlusion such as Acne vulgaris, Keratosis pilaris, Dowling-Degos disease, Hailey-Hailey disease, Mammilary fistula or follicular occlusion tetrad syndromes including Acne conglobata, Pilonidal sinus disease, Dissecting cellulitis of the scalp and Hidradenitis suppurativa for which the same tissues are responsible for disease development; or it may be for esthetic or cosmetic use for removing undesired hair from the human body.

The invention further relates to compositions as defined for use as a medicament.

### Short description of the figures

Figure 1: Figure 1 shows a screening assay to identify enzymes that release hair from skin
Figure 2: Figure 2 shows the quantification of hair release from skin after topical application of enzyme compositions
Figure 3: Figure 3 shows that glutamyl endopeptidase potently disrupts human hair ORS tissue
Figure 4: Figure 4 shows that glutamyl endopeptidase potently and selectively hydrolyse ORS-derived keratins
Figure 5: Figure 5 shows that glutamyl endopeptidase selectively disrupts healthy human ORS tissue
Figure 6: Figure 6 shows that glutamyl endopeptidase selectively disrupts ORS tissue in lesional Hidradenitis suppurativa skin
Figure 7. Figure 7 shows that glutamyl endopeptidase facilitates enhanced follicular enzyme delivery

### Detailed description of the invention

According to the invention, dermatoses such as follicular skin conditions can be treated with a composition comprising one or more enzymes capable of catalysing the hydrolysis of keratinocyte effector molecules such as protein keratins that support the integrity of the biological barrier, function and structure of the human skin and its appendages such as the hair follicle.

Remarkably, it was discovered that enzymes capable of releasing hair from mammal skin selectively and potently catalyse the hydrolysis of type I and II keratinocyte proteins such as keratins that support the function and integrity of the biological barrier, function and structure of the human skin and its appendages such as the hair follicle, so that they can be used to treat human skin and/or remove or decrease congestion of the hair follicles to avoid dermatoses and cosmetic conditions. Thereby, said enzymes may prevent and/or reduce and/or control disease development triggered by these proteins such as keratins to cause early remission of disease states in skin including its appendages including the hair follicle by inhibiting progression and recurrence of dermatoses and their skin manifistations or other unwanted consequences without exerting significant unwanted damage.

### Enzymes

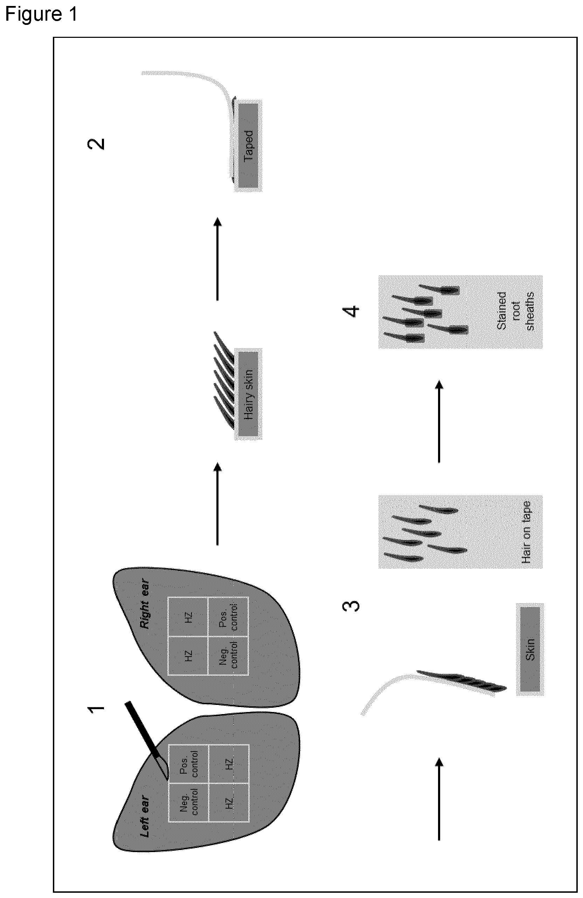

The one or more enzymes capable of catalysing the hydrolysis of keratinocyte effector molecules such as protein keratins that support the integrity of the biological barrier, function and structure of the human skin and its appendages such as the hair follicle, may in principle be any enzyme having the ability to catalyse the hydrolysis of such proteins including those classified as keratins. Proliferative or hyperproliferative keratinocyte keratins that mediate dermatoses and that support the integrity of the human hair follicle tissues surrounding the hair and facilitating the natural growth of the hair in its follicle, are typically found in the human hair anchor tissues, including the ORS or IRS or any other specifically defined functional tissue of skin or barrier breached skin with appendages, and the functional and structural protein keratins surrounding human hairs that are instrumental to previously exampled conditions or disease development. The skilled person can determine whether a given enzyme has the ability to disrupt the tissue made up of proliferative or hyperproliferative keratinocyte proteins that may be classified as keratins by simple routine experiments such as the experiment and its results disclosed in Examples 1-10 and Figures 1-7. For the purpose of this invention an enzyme is considered to have the ability to disrupt the ORS tissue of the healthy hair follicle when its activity measured on hairy pig skin as substrate relative to negative control and enzyme controls when the force reduction when plucking the hair after topical application is higher than 40%, preferable higher than 50%, preferable higher than 60%, preferable higher than 70% and most preferable higher than 80% or sufficiently high to avoid dicernable pain in humans removing hairs from skin.

Preferably, the one or more enzymes capable of catalysing the hydrolysis of keratinocyte effector molecules such as protein keratins that support the function and integrity of the biological barrier, function and structure of the human skin and its appendages such as the hair follicle, are selected among proteases including the endopeptidases, in particular glutamyl-specific proteases i.e. proteases having a high specificity to cleave peptide bonds adjacent to a glutamyl residue; and more preferred the one or more enzymes are selected among glutamyl endopeptidases that due to their selectivity towards glutamate amino acids in protein substrates and keratins may avoid signficiant collateral damage to the dermis. Example of such enzymes are Glutamyl endopeptidase II sprE from *Streptomces griseus,* V8 protease from *Staphylococcus aureus*, in particular Glutamyl endopeptidases from *Bacillus,* in particular the Glutamyl endopeptidase Serine protease bppB from *Bacillus pumilus JA16* is preferred and in particular the Glutamyl endopeptidase blaSE from *Bacillus licheniformis* is preferred.

In a preferred embodiment the one or more enzymes capable of catalysing the hydrolysis of keratinocyte effector molecules such as protein keratins that support the integrity of the biological barrier, function and structure of the human skin and its appendages such as the hair follicle, are selected among proteases, preferably glutamyl endoproteases, having at least 60% sequence identity, e.g. at least 70% sequence identity; e.g. at least 80% sequence identity; e.g. at least 90% sequence identity; e.g. at least 95% sequence identity; e.g. at least 96% sequence identity; e.g. at least 97% sequence identity; e.g. at least 98% sequence identity; e.g. at least 99% sequence identity; to the polypeptide having the sequence of SEQ ID NO: 1 or SEQ ID NO: 13. In a preferred embodiment the one or more enzymes capable of catalysing the hydrolysis of keratinocyte effector molecules such as protein keratins that support the integrity of the biological barrier, function and structure of the human skin and its appendages such as the hair follicle comprises the sequence of SEQ ID NO: 1 and/or SEQ ID NO: 13.

The one or more enzymes capable of catalysing the hydrolysis of keratinocyte effector molecules such as protein keratins that support the integrity of the biological barrier, function and structure of the human skin and its appendages such as the hair follicle may be a variant of the proteases comprising the sequence of SEQ ID NO: 1 and SEQ ID NO: 13, such as a variant comprising one or more, e.g. 1, 2, 3, 4, 5, 6, 7, 8, 9, or 10 substitutions, preferably conservative substitutions compared with SEQ ID NO: 1 or SEQ ID NO:13.

In another preferred embodiment the one or more enzymes capable of catalysing the hydrolysis of keratinocyte effector molecules such as protein keratins that support the integrity of the biological barrier, function and structure of the human skin and its appendages such as the hair follicle, are selected among proteases, preferably glutamyl endoproteases, having at least 60% sequence identity, e.g. at least 70% sequence identity; e.g. at least 80% sequence identity; e.g. at least 90% sequence identity; e.g. at least 95% sequence identity; e.g. at least 96% sequence identity; e.g. at least 97% sequence identity; e.g. at least 98% sequence identity; e.g. at least 99% sequence identity; to the polypeptide having the sequence of SEQ ID NO: 12 or SEQ ID NO: 14. In a preferred embodiment the one or more enzymes capable of catalysing the hydrolysis of keratinocyte effector molecules such as protein keratins that support the integrity of the biological barrier, function and structure of the human skin and its appendages such as the hair follicle comprises the sequence of SEQ ID NO: 12 and/or SEQ ID NO: 14. The one or more enzymes capable of catalysing the hydrolysis of keratinocyte effector molecules such as protein keratins that support the integrity of the biological barrier, function and structure of the human skin and its appendages such as the hair follicle may be a variant of the proteases comprising the sequence of SEQ ID NO: 12 and SEQ ID NO: 14, such as a variant comprising one or more, e.g. 1, 2, 3, 4, 5, 6, 7, 8, 9, or 10 substitutions, preferably conservative substitutions compared with SEQ ID NO: 12 or SEQ ID NO:14.

The skilled person can determine whether a given enzyme has the ability to disrupt the ORS tissue comprised of keratinocytes and their effector proteins that may be classified as keratins by simple routine experiments such as the experiment and its results disclosed in Example 3. Preferably the enzymes are able to obtain a force reduction of higher than higher than 40% relative to the negative control, when the positive control is significantly different from the negative control, when plucking the hair when the enzyme is applied. An enzyme is considered able to disrupt the healthy tissue when the enzyme is supplied in a concentration less than 1000 ppm, preferably less that 100 ppm and more preferably less than 10 ppm. A particularly good concentration range is between 0.01 and 1 ppm, and even more preferred is a concentration range between 0.01 and 0.05 ppm.

For skin sample analyses (Examples 6-9) the ability of enzyme compositions to disrupt the ORS tissue can be observed by histology under the microscope after enzyme incubation using 30-1000 ppm enzyme or preferably 1-10 ppm enzyme, preferably using less than 1 ppm enzyme, or even less than 0.1 ppm enzyme and more preferably using 0.01-0.1 ppm enzyme but not in the negative control buffer (no enzyme added), preferably when it visibly disrupts the ORS on plucked beard hair so that it swells significantly under the microscope after enzyme incubation using 30-1000 ppm enzyme or preferably 1-10 ppm enzyme, preferably using less than 1 ppm enzyme, or even less than 0.1 ppm enzyme and preferably using 0.01-0.1 ppm enzyme but not in the negative control buffer, or it particularly degrades the ORS tissue visibly by disrupting the epithelium using 30-1000 ppm enzyme or preferably 1-10 ppm enzyme, preferably using less than 1 ppm enzyme or even less than 0.1 ppm enzyme and preferably using 0.01-0.1 ppm enzyme but not in the negative control buffer without significantly damaging the ORS cells as observed under the microscope after enzyme incubation but not in the negative control buffer.

The skilled person can determine whether a given enzyme has the ability to disrupt the healthy ORS tissue comprising keratinocytes and their effector proteins that may be classified as keratins by simple routine experiments such as the experiment and its results disclosed in Example 6-8.

The skilled person can determine whether a given enzyme has the ability to disrupt the lesional ORS tissue comprised of keratinocytes and their effector proteins that may be classified as keratins by simple routine experiments such as the experiment and its results disclosed in Example 9.

### Pharmaceutical compositions

The pharmaceutical compositions of the invention are used by topical application to the skin area to be treated. The skilled person can determine whether a given enzyme has been delivered to the site of action in the hair follicle after topical application by simple routine experiments such as the experiment and its results disclosed in Example 10, in which enzyme facilitates gold nanoparticle delivery into the hair follicle at least 300 µm from the skin surface. An enzyme is considered delivered topically in the present invention when gold nanoparticles penetrate significantly deeper into the hair follicle as a result of enzymatic activity relative to the negative control (no enzyme in the same composition).

Thus, the composition of the invention is preferably in a form:
- that is suitable for topical application such as in the form of a cream, lotion, gel, ointment, foam, film, spray, suspensions of particles, filaments or spheres with a size of 10 to 1000 nanometers or combinations thereof that more readily and deeply enter into hair follicles and skin; or
- Of a patch that physically and/or chemically promotes enhanced ingredient and enzyme penetration of the skin to the active site optionally fitted such as an occlusive measure that promotes skin swelling or microscopic needles or similar pointy formations or a reservoir to hold composition; or
- Of a skin implant such as a physical reservoir, pump or particles placed by a device such as a needle; or other formulation or administration forms known, accepted or accepted in the future for targeted application to skin or lesions such as topical application of an active ingredient in a composition.

The composition is also preferred to be formulated as an intrafollicular, intradermal injection preferably an intralesional injection such as using a hollow needle to treat existing lesions by injecting a solution to the site of action where the causative proteins of advanced lesions with potentially lower permeability are to be hydrolysed.

Preferably, the one or more enzymes capable of catalysing the hydrolysis of keratinocyte effector molecules such as protein keratins that support the function and integrity of the biological barrier, function and structure of the human skin and its appendages such as the hair follicle are applied in an amount of 10ng to 10.000 µg enzyme protein per square centimetre skin, preferably 100 ng to 1000µg enzyme protein per square centimetre skin, more preferably 100 ng to 100 µg enzyme protein per square centimetre skin.

Preferably, the one or more enzymes capable of catalysing the hydrolysis of keratinocyte effector molecules such as protein keratins that support the function and integrity of the biological barrier, function and structure of the human skin, occluded hair follicles and skin lesions such as cysts are injected into affected skin in an amount of 10ng to 10.000 µg enzyme protein per skin area that needs to be treated, preferably 100 ng to 1000µg enzyme protein per skin lesion, more preferably 100 ng to 100 µg enzyme protein per skin area that needs to be treated.

The composition of the invention preferably comprises one or more enzymes capable of catalysing the hydrolysis of keratinocyte effector molecules such as protein keratins that support the function and integrity of the biological barrier, function and structure of the human skin and its appendages such as the hair follicle in an amount in the range of 1ng to 100 mg enzyme protein per g of the composition; or expressed in ppm defined in this invention as 1 ppm = 1 mg enzyme protein per L volume of the composition of the invention, preferably one or more enzymes capable of catalysing the hydrolysis of keratinocyte effector molecules such as protein keratins that support the function and integrity of the biological barrier, function and structure of the human skin and its appendages such as the hair follicle in an amount of 0.01 ppm to 1000 ppm of the composition, preferably 1 ppm to 100 ppm of the composition, more preferably 30 ppm to 100 ppm of the composition.

In one embodiment the composition is a pharmaceutical composition, wherein the ingredients and their concentrations are or will be pharmaceutically acceptable in accordance with relevant and current pharmaceutical regulations, or otherwise approved specifically by the regulatory authorities for the specific composition to be marketed and applied within the relevant market, condition or disease.

"Pharmaceutically acceptable ingredients" or "ingredients that are pharmaceutically acceptable" or grammatically equivalent terms are in the present specification and claims intended to mean ingredients that have the uniformness and purity required to be used as components in the manufacture of pharmaceutical compositions. The person skilled in the art knows how to select suitable ingredients that are acceptable for such use, e.g. by use of recognized pharmacopoeia such as the US Pharmacopeia or the European Pharmacopeia or equivalent legal and regulatory bodies globally.

In one embodiment, in addition to the at least one pharmaceutically acceptable ingredient additional excipients are added to the composition.

In one embodiment, the composition further comprises other pharmaceutically active molecules.

In one embodiment, the composition further comprises pharmaceutically active molecules that reduce pain.

In one embodiment, the composition further comprises pharmaceutically active molecules that reduce inflammation.

In one embodiment, the composition further comprises pharmaceutically active molecules that reduce hair growth.

In one embodiment, the composition is a semisolid composition such as an emulsion.

In one embodiment, the composition is a suspension comprising a liquid or semisolid composition with dispersed solid or semisolid particles, filaments or mixtures thereof. Preferably, the particles have an average size of 10-10,000 nm, preferably 50-2,000 nm, particularly 100-800 nm.

In one embodiment, the composition further comprises pharmaceutically active molecules that benefit from being topically delivered.

### Additional composition excipients

The composition of the invention comprises one or more enzymes capable of catalysing the hydrolysis of keratinocyte effector molecules such as protein keratins that support the function and integrity of the biological barrier, function and structure of the human skin and its appendages such as the hair follicle, and one or more further ingredients selected among: water, pH adjusting agents, preservatives, protein denaturants such as chaotropic agents, organic salts, reducing agents, diluents, ionic strength adjusters, surfactants, alcohols, organic salts, chelators, pH adjustment compounds, enzyme co-factors, waxes, composition and enzyme stabilizers, essential oils, lipids including oils and waxes, structural and active polymers, and in combination with anti-inflammatory and pain relief compounds, skin peeling agents, keratinolytic agents such as, healthy microbiome promoting agents. Preferably the one or more further ingredients are selected among: enzyme activating agents such as Calcium salts, pH buffer salts, stabilizing agents such as polyols and sugars, wetting agents such as surfactants, penetration enhancing ingredients such as chaotropic and reducing agents and water.

In one embodiment, the composition has additional excipients that lower the water activity by being present in the composition in 1 to 99% concentration, preferably in 1 to 80% concentration, preferably in 1 to 60% concentration, more preferably in 1 to 40%, because it lowers microbial growth that could spoil the composition and because it increases the stability of enzymes such as proteases as their activity depend on water. Preferably these excipients are selected from polyols as glycerol and polyethylene glycols.

In one embodiment, the composition further comprises a surfactant. The hydrophilic lipophilic balance (HLB) of a surfactant is a measure of the degree to which it is more hydrophilic or more lipophilic and known to a person skilled in the art.

In one embodiment, the surfactant is cationic.

In another embodiment, the surfactant is anionic such as sodium lauryl sulphate to ensure wetting of compositions containing water and enhance skin swelling that promotes permeability of molecules towards external compounds.

In another embodiment the surfactant is a nonionic surfactant with low HLB such as sorbitan laurate such as Span 20 to ensure wetting of water-containing compositions and facilitate delivery through hydrophobic barrier such as follicular sebum. Preferably the nonionic surfactant has HLB values between 0 and 20, more preferably HLB values between 5 and 12. Preferably the nonionic surfactant has an HLB between 5 and 18 such as 0.001-10% Triton X-100. Preferably the surfactant has HLB between 5 and 12, such as a 0.01-1% Triton X-100. Another suitable nonionic surfactant is a nonionic surfactant with low HLB such as sorbitan laurate such as 0.1-5% Span 20. Preferably the surfactant is a nonionic surfactant with low HLB such as sorbitan laurate such as 0.5-2% Span 20.

In one embodiment, different surfactants are mixed to lower the free monosurfactant concentration that may disturb the stability of proteins such as enzymes. A good mixture is a higher HLB 8 to 20, nonionic surfactant such as 0.01-1% Triton X-100 with a nonionic surfactant with low HLB 0 to 12 such as sorbitan laurate such as 0.1-5% Span 20. Another good mixture is combining a nonionic and anionic surfactants with low water activity.

In one embodiment, the composition further comprises a buffer salt such as 10-1000 mM Tris/HCl that ensures a stable pH in a range where the one or more enzymes in the composition are most active. In a preferred embodiment, the composition comprises a buffer salt that allows the composition to limit potential skin irritation close to the physiological conditions such as 25-200 mM Tris/HCl that ensures a stable pH in a range where the one or more enzymes in the composition are most active.

In one embodiment, the composition further comprises a salt that allows the composition to limit potential skin irritation close to the physiological conditions such as 0.01-2% NaCl. In a preferred embodiment, the composition comprises a salt to adjust the ionic strength to a physiologically more optimal concentration such as 0.5-1.5% NaCl that ensures an ionic strength in a range where the one or more enzymes in the composition are most active.

In one embodiment, the composition further comprises a thickener.

In one embodiment, the composition further comprises a preservative such as benzoate, propionate or sorbates to avoid microbial growth that could spoil the composition. Preferably sorbates such as 0.001-10% potassium sorbate. In a preferred embodiment, the composition comprises a preservative such as 0.01-2% potassium sorbate.

In one embodiment, the composition comprises a soluble calcium salt such as 0.1-100 mM CaCl₂, because it is an enzyme activating and stabilizing co-factor. In a preferred embodiment, the composition comprises a soluble calcium salt such as 1-10 mM CaCl₂ to stabilize the enzymes that benefit.

In one embodiment, the composition comprises a chaotropic agent such as 0.01-8 M urea, because it partly can solubilize or facilitate swelling of skin protein thus making it more susceptible to enzyme activity. In a preferred embodiment, the composition comprises 0.1-4 M urea to facilitate skin swelling and increased dissolving of keratin.

In one embodiment, the composition comprises a reducing agent such as 0.1-1000 mM dithiothreitol or glycolic acid, because it disrupts cystine bridges that crosslinks proteins by chemical reduction that may be substrates for enzymes thus making them more susceptible to enzyme activity. In a preferred embodiment, the composition comprises 1-20 mM dithiothreitol or glycolic acid to facilitate reduction and breaking of disulphide bridge bonds in keratins to increase enzyme substrate accessibility.

In one embodiment, the composition comprises an alkaline salt such as 1-1000 mM NaOH to allow the pH to be adjusted which facilitates enzyme activity, stability and substrate dissolution. In a preferred embodiment, the composition comprises NaOH to allow an alkaline pH composition that facilitates skin swelling and increased keratin dissolution.

In one embodiment, the composition comprises an acidic salt such as 1-1000 mM HCl to allow the pH to be adjusted which facilitates enzyme activity, stability and substate dissolution. In a preferred embodiment, the composition comprises HCl to allow an acidic composition that facilitates enzyme activity, stability and physiologically tolerated pH.

In one embodiment, the composition comprises chemical and biological agents in pharmaceutically or cosmetically accepted concentrations used to topically treat hair follicle-linked conditions such as Hidradenitis suppurativa and Acne forms such as currently commercially used chemical and biological agents such as benzoyl peroxide, 1,3-benzenediol, azelaic acid, salicylic acid, retinol hormones and its derivatives and preferably any compound used to treat Hidradenitis suppurativa and Acne forms in a pharmaceutical and cosmetic composition.

In one embodiment, the composition comprises sufficient hyaluronic acid to facilitate enhanced enzyme delivery to skin and its hair follicles.

In one embodiment, the composition comprises a peptide that facilitates enhanced enzyme delivery to skin and its hair follicles.

### Cosmetic compositions

In another embodiment the composition is a cosmetic composition, where the ingredients are of a suitable standard for cosmetic compositions and can be approved by regulatory bodies e.g. by existing or obtaining approval into the cosmetic ingredient database of The European Commission.

Cosmetically acceptable ingredients or ingredients of a suitable standard for cosmetic compositions are in the present specification and claims intended to mean ingredients that have the uniformness and purity required to be used as components in the manufacture of cosmetic compositions. The person skilled in the art knows how to select suitable ingredients that are acceptable for such use, e.g. by use of recognized standards for the cosmetic industry and its legislation by e.g. The European Commission or equivalent institutions depending on the area of legislation. In one embodiment the cosmetic composition comprising the same or related, but cosmetically acceptable ingredients and concentrations as pharmaceutical compositions.

In a particularly suited embodiment, the invention relates to:
A composition comprising one or more glutamyl endopeptidases capable of catalysing the hydrolysis of keratinocyte effector molecules such as protein keratins that support the function and integrity of the human skin and its appendages such as the hair follicle that are supported by keratinocytes to maintain the biological barrier, function and structure, where the composition is:
a. a pharmaceutical composition, comprising at least one pharmaceutically acceptable ingredient selected from surfactants that open up protein substates to enzyme activity, enzyme activity enhancing and stabilizing agents and agents enabling enzymes to penetrate the outer layers of the skin and the hair follicle and the site of action to facilitate the use and purpose of the composition in addition to the one or more glutamyl endopeptidases capable of catalysing the hydrolysis of keratinocyte effector molecules such as protein keratins that support the function and integrity of the human skin and its appendages such as the hair follicle; or
b. a cosmetic composition, comprising at least one cosmetically acceptable ingredient selected from surfactants that open up protein substates to enzyme activity, enzyme activity enhancing and stabilizing agents and agents enabling enzymes to penetrate the outer layers of the skin and the hair follicle and the site of action to facilitate the use and purpose of the composition in addition to the one or more glutamyl endopeptidase capable of catalysing the hydrolysis of keratinocyte effector molecules such as protein keratins that support the function and integrity of the human skin and its appendages such as the hair follicle.

Preferred glutamyl endopeptidases in the above embodiment are glutamyl endopeptidases from *Bacillus,* the glutamyl endopeptidase Serine protease bppB from *Bacillus pumilus* JA16 and the Glutamyl endopeptidase blaSE from *Bacillus licheniformis.*

In general for all embodiments a glutamyl endopeptidase is an endopeptidase with a glutamyl endopeptidase ratio (GR) > 5, calculated as GR = activity on Suc-AAPE-pNA /activity on Suc-AAP(not)E-pNA with the highest activity, where Suc-AAP(not)E-pNA is one of the following Suc-AAPA-pNA, Suc-AAPD-pNA, Suc-AAPF-pNA, Suc-AAPI-pNA, Suc-AAPK-pNA, Suc-AAPL-pNA, Suc-AAPM-pNA, Suc-AAPR-pNA or Suc-AAPV-pNA.

It is preferred that an aquous or emulsion composition that is applied topically or injected into lesional skin further comprises at least one surfactant preferable selected among anionic, nonionic and mixtures thereof that is compatible with enzyme stability and activity and facilitates enzyme penetration of the outer layers of the skin and delivery to the hair follicle and site of action to facilitate the use and purpose of the composition.

It is preferred that a suspension composition that is applied topically or injected into lesional skin comprises a liquid or semisolid composition with dispersed solid or semisolid particles, filaments or mixtures thereof. Preferably, the particles have an average size of 200-800 nm for topical follicular delivery and 80-10.000 nm for injectable compositions.

Preferably the surfactants that open up protein substates to enzyme activity are selected from Span 20, Span 40, Span 60, Span 80 and Brij 72.

Preferably the enzyme activity enhancing and stabilizing agents are selected from glycerol, sugars and propylene glycol, calcium salts and enzyme inhibitors.

Preferably the agents enabling enzymes to penetrate the outer layers of the skin and the hair follicle and enter the site of action to facilitate the use and purpose of the composition are selected from reducing agents such as glycolic acid, chaotropic agents such as urea and wettings agents such as sorbinan laurate.

Preferably the compositions according to the invention are either applied:
- topically because it could provide both safe, preventive and therapeutic benefits, is commercially efficient to produce and is well know to regulatory bodies ; or
- by microneedle patch because it is patient/customer-friendly/convenient/limits enzyme to only the targeted skin area unlike a topical cream and pressure on sore skin; or
- by a hypodermic needle injection to treat isolated, injectable lesions/cysts before they may grow or spread further.

### Use of compositions for treatment

The compositions of the invention are used to treat dermatoses and hair follicle-related conditions in a human having the need for such treatment.

The term "hair follicle-related condition" is in the application intended to mean any condition related to hair follicles, its tissues, cells, intra- and extracellular proteins and its modifications and the hair shaft and its components including proteins, carbohydrates and lipids.

In one preferred embodiment the use according to the invention include pharmaceutical treatment and/or prevention of conditions such as Hirsutism, Pseudofolliculitis, Acne vulgaris or follicular occlusion tetrad syndromes including Acne conglobata, Pilonidal sinus disease, Dissecting folliculitis of the scalp and Hidradenitis suppurativa.

Mulitple follicular conditions and diseases or follicle-linked dermatoses are suited to treat with the present invention such as excessive or pathological hair growth including Hirsutism, Hypertrichosis or Pseudofolliculitis barbae; or skin diseases that are induced by follicular hyperkeratosis and follicular occlusion; or adnexal diseases that leads to development of Acne vulgaris, Keratosis pilaris, Dowling-Degos disease, Hailey-Hailey disease, Mammilary fistula, Fox fordyce, Trichostasis spinulosa, Keratosis pilaris atrophicans faciei, Atrophoderma vermiculatum and follicular occlusion tetrad syndromes including Acne conglobata, Pilonidal sinus disease, Dissecting cellulitis of the scalp and Hidradenitis suppurativa, and inflammatory hair follicle-linked conditions including Follicular psoriasis, Pityriasis rubra pilaris, Discoid lupus, Lichen planopilaris, Hypertophic lichen planopilaris, Lichen planus follicularis tumidus, Lichen schlerosus, Lichen spinulosus, Wong-type dermatomyositis, infectious hair follicle-linked conditions including Post-Kala azar dermal Leishmaniasis, Type I reactions in borderline tuberloid leprosy, Pityriasis folliculorum; and other hair follicle-linked diseases including Nevus comedonicus, Folliculotropic mycosis fungoides and Phyrnoderma.

In another preferred embodiment the use according to the invention includes cosmetic treatment of undesired hair growth on the human body.

The compositions of the invention can be used to treat follicular dermatoses and hair follicle-related conditions by applying the composition to the skin or lesion area that is to be treated, leaving the composition on or in the skin in a holding period to allow the composition of the invention to function.

During the treatment the one or more enzymes in the composition of the invention will exert its hydrolytic activity on the proteins that may be classified as keratins produced at some point by proliferative or hyperproliferative keratinocytes with the consequence that hair is loosened, and that the hair becomes removable using significantly less force than what is necessary to remove an untreated hair from same individual, or the hair may even fall off. Furthermore, the degradation of the hair follicle tissue and its root sheath-specific keratins produced by proliferative or hyperproliferative keratinocytes surrounding the hair shaft has the consequence that the frequency and/or extent of follicle occlusion/plugging is reduced, which limits the progression of occluded hair follicles into infected or disease inflicted hair follicles. The treatment may also reduce the occlusion and potentially fully unplug and rescue hair follicles in the early stages of the diseases such as Hidradenitis suppurativa or related syndromes, thereby controlling and preventing the disease progression, recurrence and development of the early stage disease and its hallmark lesions that are followed by more severe stages of disease that bear higher burden for the patient.

The compositions of the invention may even be injected into or locally applied to body areas affected by more severe stages of the disease, where the area comprises occluded and/or infected hair follicles or skin cysts; and can in this embodiment be used to reduce or intervene with disease development to avoid further advancement of the disease in one or more areas.

The holding period should be sufficiently long to allow the one or more enzymes capable of catalysing the hydrolysis of keratinocyte effector molecules such as protein keratins that support the integrity of the biological barrier, function and structure of the human skin and its appendages such as the hair follicle and have sufficient effect. Typically, the holding period for topically applied compositions is selected in the range of 2 minutes to 24 hours, e.g. in the range of 3-60 minutes, e.g. in the range of 5-30 minutes, e.g. during overnight sleep. In some embodiments e.g. where the composition is a gel; the holding period will in practice continue until the enzyme becomes inactive, which e.g. for a gel composition will happen when the skin, mucosal or hair follicle tissue naturally desquamates and pushes out the enzyme after hours to days to a few weeks and/or when the skin is cleaned externally. In some embodiments e.g. where the composition is a medical patch or otherwise nonliquid compositions that can be fully recovered by physical peeling or washing it off; the holding period will in practice continue until the enzyme becomes inactive, which e.g. for a medical patch composition could happen after hours to multiple days. In some embodiments e.g. where the composition is an intralesional injection a holding period is not relevant.

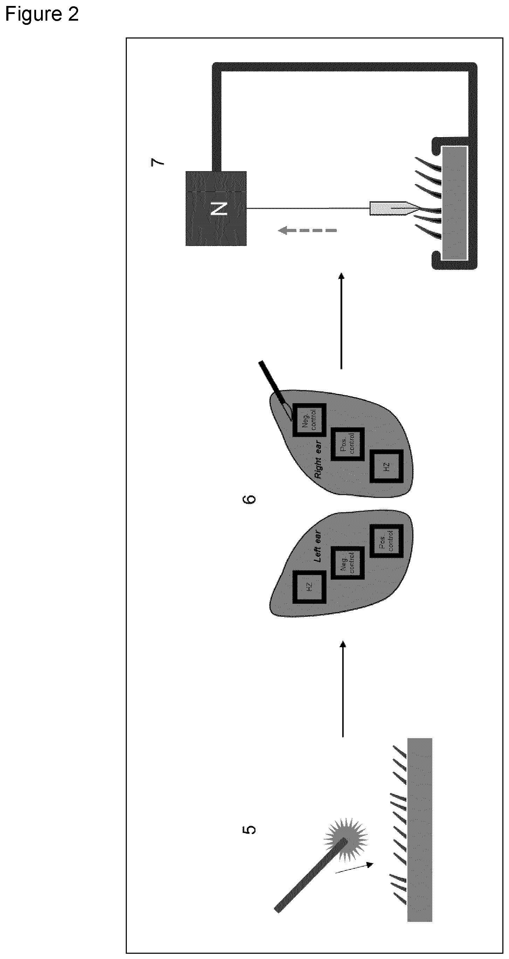

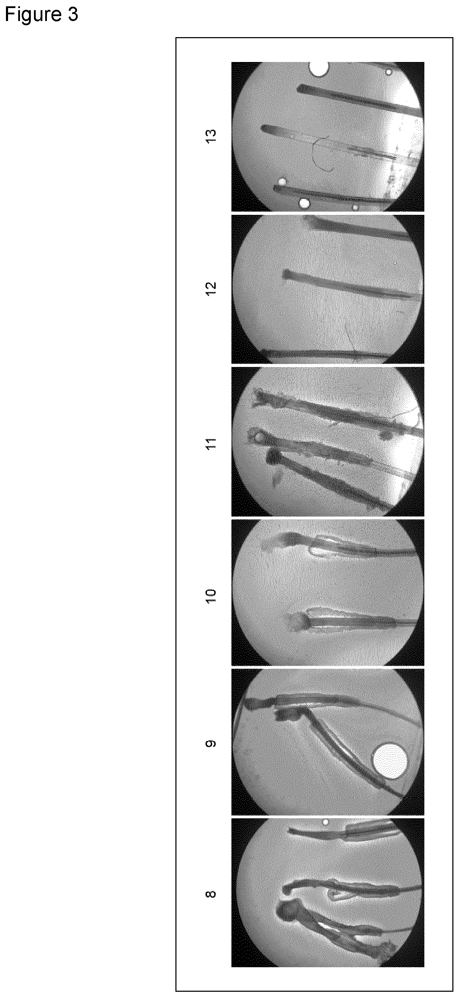

The integrity of the ORS of a hair follicle and lesional tissue that still has a hair shaft can be evaluated by the person skilled in the art using a simple technical concepts as described in Figure 2 and Example 3. The force necessary to remove a hair is a convenient functional, quantitative measure that can be used by the person skilled in the art using well known techniques described in Figure 2. When the force necessary to remove a treated hair is compared with the force necessary to remove an untreated hair, it is preferred to make the measurement on several individual hairs and calculate an average force in order to compensate for the hair to hair variation that will occur due to varying skin biology and types, its current condition and natural hair follicle cycle in which hairs are grown, shed and regrown over time. So according to the invention the force necessary to remove a treated/untreated hair should preferably be calculated as an average of at least 3 or more hairs on a skin biopsy. If fewer are available on the skin biopsy, it is preferred to calculate the average based on all available hairs on the biopsy. Preferably the force necessary to remove a treated hair in comparison with an untreated hair is reduced with at least 10%, preferably at least 20%, preferably at least 30%, preferably at least 40%, preferably at least 50%, preferably at least 60%, or at least 70%, or at least 80%, or to at least an extent that corresponds to reduced pain to pain-free hair removal, or increased hair follicle-linked disease prevention or control. An example of a method for measuring the force necessary to remove a hair is shown in Example 3. The integrity of the ORS in human skin can be further evaluated by histological imaging after incubating plucked human hairs with ORS with enzymes. The histological integrity of the ORS and the surrounding tissue is inspected by a person skilled in the art using microscopy and staining specific to tissue and cell components. Examples of methods for a person skilled in the art to evaluate differences that can be considered to be beneficial for the purpose of the present invention involving compositions to treat follicular dermatoses and hair follicle-linked conditions are shown in Examples 1-10 and Figures 3-7.

In one embodiment the compositions of the invention are used for the treatment of Hirsutism and Hypertrichosis. In this embodiment the invention provides an efficient treatment that allows the removal of excessive hair shafts including its root from the affected skin area creating a longer lasting effect with less or no pain.

In another embodiment the composition of the invention is used for a treatment of Pseudofolliculitis. In this embodiment the invention provides an efficiently treatment that allows the removal of excessive hair from affected skin area particularly of the face and neck or reduces irritation of the skin where hairs are misshaped and damage the skin, which is likely caused by dysfunction of the hair follicle anchor tissues, cells and its proteins.

In another embodiment the composition of the invention is used for the treatment of Acne vulgaris. In this embodiment the invention provides an efficient treatment that allows relieve of comedones, papules, nodules, cysts, postules, lesions, and the removal of excessive hair from affected skin area with a low risk of skin irritation.

In another embodiment the composition of the invention is used for a treatment of Keratosis pilaris in which occluded hair follicles generate itchy and widespread nodules.

In another embodiment the composition of the invention is used for a treatment of the follicular hyperkeratosis and occlusion-linked diseases such as Dowling-Degos disease, Hailey-Hailey disease, Mammilary fistula.

In another embodiment the composition of the invention is used for a treatment of Hidradenitis suppurativa and the other follicular tetrad or follicular occlusion diseases that rely on the same hair follicle keratin-linked developments for the condition to develop. In this embodiment the invention is particularly suited for treating the earlier stages of lesion development where the composition of the invention is used to prevent or reduce occlusion of hair follicles before late stage and more severe symptoms presents. Since all patients with Hidradenitis suppurativa and related diseases as described have recurring lesions that are a result of hair follicles cycling through growth stages, the invention benefits all stages of condition or disease severity.

In another embodiment the composition of the invention is used for a treatment of inflammatory hair follicle-linked conditions including Follicular psoriasis, Pityriasis rubra pilaris, Discoid lupus, Lichen planopilaris, Hypertophic Lichen Planopilaris, Lichen Planus Follicularis Tumidus, Lichen Schlerosus, Lichen Spinulosus, Wong-type dermatomyositis, infectious hair follicle-linked conditions including Post-Kala Azar dermal Leishmaniasis, Type I Reactions in borderline tuberloid leprosy, Pityriasis Folliculorum, and other hair follicle-linked disesases including Nevus Comedonicus, Folliculotropic Mycosis Fungoides and Phyrnoderma.

The invention is further described and supported by the following examples that are provided to illustrate the invention and should not be considered limiting in any way.

### Examples

### Example 1: Screening of endopeptidases for ORS tissue activity by hair release

The present example describes an assay for screening a panel of different endopeptidases for their ability to release hairs from skin, to provide a measure of catalysis of the hydrolysis of proteins such as hair anchoring keratins that support the function and integrity of the hair follicle.

The experimental design is described in Figure 1. Pig ears are good physiological models of hairy human skin, but the inherent biological variation needs to be controlled. Ears were sampled postmortem from a single supplier of landrace pigs bred for animal experimentation, thoroughly rinsed with water, placed pairwise in airtight plastic bags and immediately frozen at -20°C. Ears were used for experiments less than one month after freezing. For each enzyme experiment, one pair of frozen ears was thawed over a period of approximately 1 hour in 250 mL physiological 150mM NaCl (20°C) in a plastic bag to ensure that the skin was fully covered during thawing. The salt solution was discarded, and the ears were washed thoroughly with 100 mL shampoo solution (4 mL MacUrth mild organic shampoo with disodium lauryl sulfosuccinate (anionic) and coco glucoside (nonionic) surfactants + 96 mL water) for two minutes to remove dirt and reduce the microbiome. The ears were rinsed in tap water and dried with paper towels before dissection of the skin.

To best separate biological variation from enzyme effects, mirrored positions on the ear pair used for each experiment were used for reference and enzyme samples (1). In order to account for differences in absolute effect between pairs, negative and positive controls were included on all ears. The positive control was Glutamyl endopeptidase blaSE from *Bacillus licheniformis* that showed most effect during the design phase of the assay allowing this datapoint to validate that the biological sample was reliable and consistent between ear pairs. 4 skin pieces of 20x20 mm per ear = 8 pieces per pair in each experiment were carefully cut out of the dorsal side of the ears and the skin was carefully removed from the cartilage by cutting next to the cartilage surface with a sharp scalpel.

The assay was run at skin surface temperature of 30°C using 25 mM Tris/HCl, 150 mM NaCl, 5 mM CaCl₂, 2 mM potassium sorbate, 1% Span 20 buffered to neutral pH=7.4 (HZ Buffer and negative control). These conditions were selected respectively to be suited for pharmaceutical and cosmetic compositions, stabilize the enzymes by calcium ions, reduce microbial growth during the experiment, facilitate wetting and limit irritation.

The 11 endopeptidases screened are listed in Table 1 and represent a broad diversity of endopeptidase classes that have different proteolytic specificities publicly annotated in the UniProt or MEROPS databases. They were separately purified by column chromatography to a purity where a single band could be observed after SDS-PAGE separation and Coomassie Blue staining.

**Table 1**

| Enzyme | Endopeptidase | SEQ ID NO: | Public reference |
|---|---|---|---|
| HZ-2 | Glutamyl endopeptidase blaSE from *Bacillus licheniformis* | 1 | Geneseqp:BGC99321 |
| HZ-3 | Subtilisin Carlsberg from *Bacillus licheniformis* | 2 | Uniprot:B0FXJ2 |
| HZ-4 | Lysyl endopeptidase from *Achromobacter lyticus* | 3 | Uniprot:P15636 |
| HZ-6 | Thermostable alkaline protease BH0855 from *Bacillus halodurans* | 4 | Uniprot:P41363 |
| HZ-8 | Metalloprotease from *Saccharothrix variesporea* | 5 | Uniprot:A0A495XKH6 |
| HZ-9 | Bacillolysin npr from *Bacillus amyloliquefaciens* | 6 | Uniprot:P06832 |
| HZ-10 | Subtilisin 309 variant from *Bacillus clausii* | 7 | Geneseqp:BEE82947 |
| HZ-11 | Serine protease from *Nocardiopsis* sp NRRL 18262 | 8 | Geneseqp:BDA19904 |
| HZ-18 | Snapalysin protease snpA from *Streptomyces coelicolor* | 9 | Uniprot:P0A3Z7 |
| HZ-19 | Trypsin from *Fusarium oxysporum* | 10 | Uniprot:P35049 |
| HZ-32 | Deuterolysin protease Ta1 from *Thermoascus aurantiacus* | 11 | Geneseqp:AZS91964 |

Two skin pieces were incubated as negative controls (2400 µl HZ Buffer per skin piece), two skin pieces were incubated as positive controls (2400 µl HZ Buffer with 100 ppm HZ-2 per skin piece) and four skin pieces were incubated as endopeptidase (HZ in (1)) incubations (2400 µl HZ Buffer with 100 ppm endopeptidase per skin piece). The skin pieces were incubated for 3 hours at 30°C. The solutions were discarded, and the skin pieces were rinsed thoroughly with tap water and dried using paper towels and leaving them under a fume hood for 1 hour.

A 50x25 mm piece of sports tape (Sportsdoc Medical Pro Deluxe, 25mm. From Svea medical sports AB, Sweden) was gently placed onto each skin piece oriented in the same direction as the direction of the hairs and pressed firmly applying enough force to ensure contact between hairs and adhesive (2). The tape was pulled off in one movement in a direction opposite of the natural direction of the hairs (3). The adhesive of the sports tape is so strong that hairs would either break, leaving the hair roots in the skin, or the tape would pull out the hairs with the root sheaths intact. After tape stripping, the tape was stained with 1% para-dimethylamino cinnamaldehyde (DMACA) in 0.5 M HCl, which stains the IRSs bright red (4). The number of red IRSs and the length of them are convenient means to identify the ability of an endopeptidase to catalyze the hydrolysis of proteins such as hair anchoring keratins that support the function and integrity of the hair follicle. After taking photos, the IRSs were evaluated by counting the numbers of red IRS and by measurement of the total length IRS present on the tape using a drawing software (OpenOrienteering Mapper) using the width of the tape to normalize the data. Results are listed in Table 2 below. To account for ear pair to ear pair variation, the relative number of IRS and the relative total length of IRS were calculated as: ((enzyme - neg. control)/(pos. control - neg. control))*100%.

**Table 2:**

| Enzyme | No. IRS (Neg. control) | No. IRS (Pos. control) | No. IRS (Enzyme) | Relative no. IRS (%) | mm IRS (Neg. control) | mm IRS (Pos. control) | mm IRS (Enzyme) | Relative mm IRS (%) |
|---|---|---|---|---|---|---|---|---|
| HZ-2 | 5.5 | 52 | 54.75 | **106** | 4.6 | 59.15 | 64.025 | **109** |
| HZ-3 | 1 | 34 | 2 | **3** | 1.3 | 39.15 | 1.75 | **1** |
| HZ-4 | 0 | 27 | 12.25 | **45** | 0 | 28.3 | 14.55 | **51** |
| HZ-6 | 0 | 40 | 3 | **8** | 0 | 46.75 | 4.0 | **9** |
| HZ-8 | 4 | 25 | 4 | **0** | 4.65 | 22.85 | 4.875 | **1** |
| HZ-9 | 0 | 53 | 7 | **13** | 0 | 61.8 | 7.875 | **13** |
| HZ-10 | 0 | 37.5 | 11 | **29** | 0 | 36.3 | 10.725 | **30** |
| HZ-11 | 0 | 17.5 | 6.75 | **39** | 0 | 18.35 | 5.875 | **32** |
| HZ-18 | 3 | 50 | 3 | **0** | 2.75 | 53.95 | 3.0 | **0** |
| HZ-19 | 0 | 40.5 | 7.25 | **18** | 0 | 42.05 | 7.5 | **18** |
| HZ-32 | 3 | 80 | 6.25 | **4** | 2.05 | 87.90 | 5.525 | **4** |

The Glutamyl endopeptidase blaSE from *Bacillus licheniformis* was identified as the best performing endopeptidase, of the eleven tested endopeptidases, that most effectively catalyzes the hydrolysis of the anchoring proteins such as keratins that support the function and integrity of the hair follicle.

In order to find out if glutamyl endopeptidases are especially suited for catalysis of the keratins that support the function and integrity of the hair follicle three glutamyl endopeptidases from other organisms (see Table 3) were studied as described above.

**Table 3:**

| Enzyme | Endopeptidase | SEQ ID NO: | Public reference |
|---|---|---|---|
| HZ-13 | Glutamyl endopeptidase II sprE from *Streptomyces griseus* | 12 | Uniprot:B1VZ60 |
| HZ-15 | Serine protease bppB from *Bacillus pumilus* JA16 | 13 | Uniprot: Q2HXL7 |
| HZ-35 | V8 protease from *Staphylococcus aureus* | 14 | Uniprot:P0C1U8 |

The results of the three additional glutamyl endopeptidases listed in Table 3 and tested in the above assay are shown in Table 4 below.

**Table 4:**

| Enzyme | No. of IRS (Neg. control) | No. of IRS (Pos. control) | No. of IRS (Enzyme) | Relative no. of IRS (%) | mm IRS (Neg. control) | mm IRS (Pos. control) | mm IRS (Enzyme) | Relative mm IRS (%) |
|---|---|---|---|---|---|---|---|---|
| HZ-13 | 0 | 44 | 17.5 | **40** | 0 | 46.05 | 20.25 | **44** |
| HZ-15 | 0 | 33 | 46 | **139** | 0 | 40.2 | 53.45 | **133** |
| HZ-35 | 0 | 26 | 11.5 | **44** | 0 | 26.05 | 13.3 | **51** |

From the above tables it can be seen that glutamyl endopeptidases are the best suited endopeptidases for catalysis of the hydrolysis of proteins such as hair anchoring keratins that support the function and integrity of the hair follicle.

### Example 2: Definition of glutamyl endopeptidase activity.

The present example describes an assay for assessing whether an endopeptidase is a glutamyl endopeptidase in the context of the present invention. Glutamyl endopeptidases are endopeptidases that cleave on the carboxy-terminal side of a glutamic acid residue (or an aspartic acid residue in phosphate buffers), i.e., they have a preference for negatively charged amino acid residues in the P1 position of the substrate. The following assay was used to test whether the endopeptidases that provided significant response in Example 1 included in SEQ NO. 1-4, 7-8, 10, and 12-14 listed in Table 1 and Table 3 used in the present invention are glutamyl endopeptidases.

20µl of each endopeptidase was diluted 0.01% Triton X-100, placed in a well in a microtiter plate at 25°C and assayed using the following commercially available substrates (Bachem AG, Bubendorf, Switzerland): Suc-AAPA-pNA (Bachem 4015680), Suc-AAPD-pNA (Bachem 4018122), Suc-AAPE-pNA (Bachem 4017343), Suc-AAPF-pNA (Bachem 4002299), Suc-AAPI-pNA (Bachem 4017698), Suc-AAPK-pNA (Bachem 4017329), Suc-AAPL-pNA (Bachem 4003646), Suc-AAPM-pNA (Bachem 4006760), Suc-AAPR-pNA (Bachem 4017320), Suc-AAPV-pNA (Bachem 401767).

The reactions were started by adding 200µl pNA substrate (50mg dissolved in 1.0ml DMSO and further diluted 75x with 50mM Tris/HCl, 0.01% Triton X-100, pH 8.0). The microtiter plate was read in a VERSAmax microtiter reader from Molecular Devices and the initial increase in OD405 was the measure of endopeptidase activity. If a linear plot was not achieved in the 4 minutes of measuring time, the endopeptidase was diluted, and the assay was repeated.

The results of the ten endopeptidases tested in the above assay are shown in Table 5 below. The data corresponds to the relative activities for each endopeptidase on the ten different Suc-AAPX-pNA substrates (X = different amino acids), i.e., the activity of the specific Suc-AAPX-pNA substrate divided by the activity of the Suc-AAPX-pNA substrate of the ten Suc-AAPX-pNA substrates with the highest activity. The dilutions of the endopeptidases were accounted for in the calculations.

**Table 5:**

| | HZ-2 | HZ-3 | HZ-4 | HZ-6 | HZ-10 | HZ-11 | HZ-13 | HZ-15 | HZ-19 | HZ-35 |
|---|---|---|---|---|---|---|---|---|---|---|
| **Glutamyl endopeptidase ratio (GR)** | 309 | 0.003 | <0.001 | 0.008 | 0.003 | <0.001 | 6 | 234 | <0.001 | 20 |
| Suc-AAPA-pNA | 0.0001 | 0.0222 | 0.0005 | 0.0880 | 0.0566 | 0.0001 | 0.0000 | 0.0000 | 0.0000 | 0.0004 |
| Suc-AAPD-pNA | 0.0020 | 0.0008 | 0.0006 | 0.0053 | 0.0001 | 0.0028 | 0.1584 | 0.0043 | 0.0000 | 0.0496 |
| Suc-AAPE-PNA | 1.0000 | 0.0033 | 0.0000 | 0.0083 | 0.0025 | 0.0004 | 1.0000 | 1.0000 | 0.0001 | 1.0000 |
| Suc-AAPF-pNA | 0.0032 | 1.0000 | 0.0041 | 0.5904 | 1.0000 | 1.0000 | 0.0000 | 0.0000 | 0.0001 | 0.0006 |
| Suc-AAPI-pNA | 0.0000 | 0.0005 | 0.0017 | 0.0005 | 0.0003 | 0.0004 | 0.0000 | 0.0000 | 0.0000 | 0.0000 |
| Suc-AAPK-pNA | 0.0001 | 0.0155 | 1.0000 | 0.0038 | 0.0011 | 0.0778 | 0.0000 | 0.0003 | 0.8404 | 0.0011 |
| Suc-AAPL-pNA | 0.0027 | 0.8067 | 0.0019 | 1.0000 | 0.1701 | 0.1851 | 0.0000 | 0.0005 | 0.0000 | 0.0066 |
| Suc-AAPM-pNA | 0.0008 | 0.2818 | 0.0000 | 0.8496 | 0.8609 | 0.0003 | 0.0001 | 0.0000 | 0.0000 | 0.0000 |
| Suc-AAPR-pNA | 0.0001 | 0.0190 | 0.0005 | 0.0069 | 0.0002 | 0.1725 | 0.0001 | 0.0000 | 1.0000 | 0.0000 |
| Suc-AAPV-pNA | 0.0001 | 0.0007 | 0.0007 | 0.0008 | 0.0001 | 0.0121 | 0.0000 | 0.0000 | 0.0000 | 0.0005 |

It can be observed in Table 5 that the Glutamyl endopeptidase blaSE from *Bacillus licheniformis* (HZ-2), Glutamyl endopeptidase II sprE from *Streptomyces griseus* (HZ-13), Serine protease bppB from *Bacillus pumilus* JA16 (HZ-15) and V8 protease from *Staphylococcus aureus* (HZ-35) have the highest activity on the Suc-AAPE-pNA substrate, whereas they have fairly low relative activities towards other substrates. Consequently, these endopeptidases are considered to be glutamyl endopeptidases. To assess whether an endopeptidase is a glutamyl endopeptidase, we have defined a glutamyl endopeptidase ratio (GR) calculated as: GR = activity on Suc-AAPE-pNA / Suc-AAP(not)E-pNA with highest activity.

A glutamyl endopeptidase according to the present invention is defined as an endopeptidase with a glutamyl endopeptidase ratio (GR) > 5.

### Example 3: Quantification of hair releasing effect by topical treatment with endopeptidases

The present example describes an assay for quantification of hair releasing effect by selected endopeptidases by measuring the force to pull out hairs after topical application of enzyme compositions on hairy pig skin.

To separate biological variation between skin samples from enzyme effects, the same considerations as described in Example 1 applies in the present example with the following additional considerations; the outer skin layer was perforated (5), a different ear positioning was used (6) and molds were glued to the skin to ensure strictly topical interaction.

Figure 2 provides a schematic overview of the experimental design. To account for variations in the thickness of the outermost layers of the pig ear skin, and to increase deep skin side follicular delivery of endopeptidases, the dorsal side of the ears were treated with a derma roller (Argador Derma Roller system from RoHs with 540x1.5mm micro needles approved in the European Union for cosmetic use) by rolling the skin using the Derma Roller 4 times over the skin vertically, horizontally and the two diagonals, using a firm pressure on the roller to ensure that the skin was evenly perforated. To define confined 18x18 mm skin areas for enzyme treatments, three polymethylmethacrylate (PMMA) square molds were glued on mirrored positions on the dorsal side of each ear using an ethyl cyanoacrylate glue.

Physiological temperature, pH, and salt concentration of compositions were the same as in Example 1 plus 10 mM DTT (HZ Buffer DTT). The six PMMA squares on the ear pair were used for three different types of treatments: negative control, positive control and endopeptidase samples (HZ in (6)). Nine endopeptidases including SEQ ID NO: 1, 3, 6-8, 10, and SEQ ID NO: 12-14 listed in Table 1 and 3 that gave a positive response in Example 1 were included in the present example.

Two squares were negative controls (600 µl HZ Buffer DTT per square), two squares were positive controls (600 µl HZ Buffer DTT and 100 ppm HZ-2 per square) and two squares were endopeptidase sample treatments (600 µl HZ Buffer DTT and 30 ppm to 100 ppm endopeptidase per square). The skin pieces with squares and enzyme were stored horizontally for approximately 20 hours at 4°C leaving the ear intact. Subsequently, the skin pieces were incubated for 4 hours at 30°C equal to skin surface temperature.

The solutions of the squares were discarded, and the squares were removed from the skin by cutting with a scalpel along the inner sides of the squares to release hairs from the adhesive of the glue. The treated skin areas were carefully washed in deionized water and the skin pieces were dried carefully with paper towel. Finally, the force required to pull out individual hairs from each skin piece after enzyme incubation was measured by fixing them to a Sauter FK 10 Digital Force Gauge connected to a spindle motor with a string and a clamp that moved vertically (7).

### Results:

An overview of the results is shown in Table 6 below. The force reduction relative to negative control was calculated as: ((neg. control - enzyme)/(neg. control))*100%. The force reduction compared to positive control was calculated as: ((neg. control - enzyme)/(neg. control - pos. control))*100%. For each skin sample the force of at least 7 and maximum 10 individual hairs per sample were measured and an average were calculated for the duplicate samples of each treatment within the ear pair. Due to ear pair to ear pair variation, data for Force reduction compared to pos. control were only accepted and presented in the table below if the positive control (100 ppm HZ-2) was considered significantly lower than the negative control using a Student's t-test with a p-value less than or equal to 0.05.

**Table 6:**

| **Enzyme** | **Conc. (ppm)** | **Enzyme (N)** | **Neg. control (N)** | **Pos. control = 100 ppm HZ-2 (N)** | **Enzyme force reduction compared to neg. control *: p ≤ 0.05** | **Enzyme force reduction compared to pos. control (100 ppm HZ-2)** |
|---|---|---|---|---|---|---|
| HZ-2 | 30 | 0.114 | 0.255 | 0.089 | 55%* | 85% |
| HZ-4 | 100 | 0.178 | 0.217 | 0.070 | 18% | - |
| HZ-9 | 100 | 0.266 | 0.381 | 0.106 | 30%* | 42% |
| HZ-10 | 100 | 0.150 | 0.345 | 0.108 | 56%* | 82% |
| HZ-11 | 100 | 0.195 | 0.276 | 0.182 | 29%* | 86% |
| HZ-13 | 100 | 0.285 | 0.347 | 0.147 | 18% | - |
| HZ-15 | 100 | 0.142 | 0.264 | 0.116 | 46%* | 82% |
| HZ-19 | 100 | 0.243 | 0.297 | 0.063 | 18% | - |
| HZ-35 | 100 | 0.206 | 0.304 | 0.179 | 32%* | 79% |

From Table 6, it can be seen that at 100 ppm, the Glutamyl endopeptidase blaSE from *Bacillus licheniformis* HZ-2 (positive control) reduced the force the most relative to the negative control, while HZ-10, HZ-11, HZ-15 and HZ-35 at 100 ppm could only reduce the force to 79-86% of HZ-2 at 100 ppm, which was equal to HZ-2 at only 30 ppm (85%). HZ-13 showed lower performance than expected from its performance in Example 1, but Example 4 revealed that it was not stable under the specific conditions tested in the present example.

### Example 4: Stability of endopeptidase activity.

The present example describes an assay to assess whether an endopeptidase is stable within the skin application conditions tested in the present invention. One relevant skin application is the screening assay described in Example 1. Another relevant skin application is the skin side assay described as Example 3.

Two incubation buffers were used for the present stability assay. Incubation buffer A: 25mM Tris/HCl, 150 mM NaCl, 5 mM CaCl₂, 2mM potassium sorbate, 1% Span 20, pH 7.4 (used in Example 1) and incubation buffer B: 25 mM Tris/HCl, 150mM NaCl, 5 mM CaCl₂, 2 mM potassium sorbate, 10 mM DTT, 1% Span 20, pH 7.4 (used in Example 3).

Each endopeptidase was transferred to the two incubation buffers A and B by dilution to 0.10 mg/mL (at least 20x dilution) and storing a small aliquot on ice until activity analysis. The major part of incubation buffer A dilution was incubated for 3 hours at 30°C (identical to the incubations of skin pieces in Example 1). After incubation the residual activity of the endopeptidase was determined for the 30°C incubated dilution A and for the corresponding dilution kept on ice (0°C). Example 1 stability was determined as Activity of 30°C incubated dilution A divided by Activity of 0°C incubated dilution A.

The major part of incubation buffer B dilution was incubated for 4 hours at 30°C. Since enzyme inactivation is minimal at low temperatures such as 4°C, the additional overnight storage at 4°C in Example 3 had a negligible effect on the residual activity. After incubation, the residual activity of the endopeptidase was determined. Example 3 stability was determined as Activity of 30°C incubated dilution B divided by Activity of 0°C incubated dilution A. In this way we both account for the lower stability due to DTT in the buffer and the lower stability due to the longer incubation time.

**Table 7:**

| Enzyme | SEQ ID NO: | Substrate |
|---|---|---|
| HZ-2 | 1 | Suc-AAPE-pNA |
| HZ-3 | 2 | Suc-AAPF-pNA |
| HZ-4 | 3 | Suc-AAPK-pNA |
| HZ-6 | 4 | Suc-AAPF-pNA |
| HZ-8 | 5 | Protazyme AK |
| HZ-9 | 6 | Protazyme AK |
| HZ-10 | 7 | Suc-AAPF-pNA |
| HZ-11 | 8 | Suc-AAPF-pNA |
| HZ-13 | 12 | Suc-AAPE-pNA |
| HZ-15 | 13 | Suc-AAPE-pNA |
| HZ-18 | 9 | Protazyme AK |
| HZ-19 | 10 | Suc-AAPK-pNA |
| HZ-32 | 11 | Protazyme AK |
| HZ-35 | 14 | Suc-AAPE-pNA |

Suitable substrates for each endopeptidase are listed in Table 7 using either Suc-AAPX-pNA (where X represents different amino acids in different substrates) or Protazyme AK. For Suc-AAPX-pNA subtrates, residual activities were determined by an assay identical to the activity assay described in Example 2 of the present invention. For Protazyme AK, residual activities were determined by adding 20µl endopeptidase solution (diluted in 0.01% Triton X-100) to an ice-cold mixture of 500µl Protazyme AK suspension (1 Protazyme AK tablet from Megazyme Ltd., Wicklow, Ireland suspended in 2.0ml 0.01% Triton X-100) and 500µl 50mM MOPS/NaOH, 0.01% Triton X-100, pH 7.0 in a 1.5mL Eppendorf tube. The Protazyme AK assay was started by transferring the tube to a prewarmed Eppendorf thermomixer set to 37°C. The tube was then incubated for 15 minutes on the thermomixer at 1100 rpm. The tube was transferred back to the ice-bath. 200µl cooled supernatant was transferred to a microtiter plate and OD405 was read in a VERSAmax microtiter reader from Molecular Devices. OD405 for the endopeptidase sample minus OD405 for a buffer blank was a measurement of the residual activity of the endopeptidase sample. If the OD405 measurement was above 1.5 OD405 units, the endopeptidase was diluted further, and the assay was repeated. The data correspond to the relative activities for each endopeptidase calculated as described above and listed in Table 8 taking the dilution factors into account.

**Table 8:**

| Enzyme | Example 1 stability | Example 3 stability |
|---|---|---|
| HZ-2 | 0.96 | 0.98 |
| HZ-3 | 1.05 | 1.09 |
| HZ-4 | 0.97 | 0.76 |
| HZ-6 | 0.99 | 0.75 |
| HZ-8 | 1.06 | 0.03 |
| HZ-9 | 0.98 | 0.84 |
| HZ-10 | 1.00 | 1.02 |
| HZ-11 | 0.93 | 0.19 |
| HZ-13 | 0.97 | 0.17 |
| HZ-15 | 0.96 | 1.09 |
| HZ-18 | 1.07 | 0.05 |
| HZ-19 | 1.01 | 0.87 |
| HZ-32 | 1.07 | 0.97 |
| HZ-35 | 1.04 | 1.03 |

It is seen that all enzymes were stable in conditions equivalent to Example 1. Therefore, the enzymes had been active during the whole incubation period.

It is seen that not all enzymes were stable in conditions equivalent to Example 3. This is because HZ-8, HZ-11, HZ-13 and HZ-18 had reduced activity after 4 hours at 30°C in incubation buffer B, suggesting that these endopeptidases were not 100% active during the whole incubation period. These data explain why HZ-13 underperformed as a glutamyl endopeptidase in Example 3.

### Example 4: Stability of endopeptidase activity.

The present example describes an assay to assess whether an endopeptidase is stable within the skin application conditions tested in the present invention. One relevant skin application is the screening assay described in Example 1. Another relevant skin application is the skin side assay described as Example 3.

**Table 9:**

| Enzyme | SEQ ID NO: | Substrate |
|---|---|---|
| HZ-2 | 1 | Suc-AAPE-pNA |
| HZ-3 | 2 | Suc-AAPF-pNA |
| HZ-4 | 3 | Suc-AAPK-pNA |
| HZ-6 | 4 | Suc-AAPF-pNA |
| HZ-8 | 5 | Protazyme AK |
| HZ-9 | 6 | Protazyme AK |
| HZ-10 | 7 | Suc-AAPF-pNA |
| HZ-11 | 8 | Suc-AAPF-pNA |
| HZ-13 | 12 | Suc-AAPE-pNA |
| HZ-15 | 13 | Suc-AAPE-pNA |
| HZ-18 | 9 | Protazyme AK |
| HZ-19 | 10 | Suc-AAPK-pNA |
| HZ-32 | 11 | Protazyme AK |
| HZ-35 | 14 | Suc-AAPE-pNA |

Two incubation buffers were used for the present stability assay. Incubation buffer A: 25mM Tris/HCl, 150 mM NaCl, 5 mM CaCl₂, 2mM potassium sorbate, 1% Span 20, pH 7.4 (used in Example 1) and incubation buffer B: 25 mM Tris/HCl, 150mM NaCl, 5 mM CaCl₂, 2 mM potassium sorbate, 10 mM DTT, 1% Span 20, pH 7.4 (used in Example 3).

Each endopeptidase was transferred to the two incubation buffers A and B by dilution to 0.10 mg/mL (at least 20x dilution) and storing a small aliquot on ice until activity analysis. The major part of incubation buffer A dilution was incubated for 3 hours at 30°C (identical to the incubations of skin pieces in Example 1). After incubation the residual activity of the endopeptidase was determined for the 30°C incubated dilution A and for the corresponding dilution kept on ice (0°C). Example 1 stability was determined as Activity of 30°C incubated dilution A divided by Activity of 0°C incubated dilution A.

The major part of incubation buffer B dilution was incubated for 4 hours at 30°C. Since enzyme inactivation is minimal at low temperatures such as 4°C, the additional overnight storage at 4°C in Example 3 had a negligible effect on the residual activity. After incubation, the residual activity of the endopeptidase was determined. Example 3 stability was determined as Activity of 30°C incubated dilution B divided by Activity of 0°C incubated dilution A. In this way we both account for the lower stability due to DTT in the buffer and the lower stability due to the longer incubation time.

Suitable substrates for each endopeptidase are listed in Table 9 using either Suc-AAPX-pNA (where X represents different amino acids in different substrates) or Protazyme AK. For Suc-AAPX-pNA subtrates, residual activities were determined by an assay identical to the activity assay described in Example 2 of the present invention. For Protazyme AK, residual activities were determined by adding 20µl endopeptidase solution (diluted in 0.01% Triton X-100) to an ice-cold mixture of 500µl Protazyme AK suspension (1 Protazyme AK tablet from Megazyme Ltd., Wicklow, Ireland suspended in 2.0ml 0.01% Triton X-100) and 500µl 50mM MOPS/NaOH, 0.01% Triton X-100, pH 7.0 in a 1.5mL Eppendorf tube. The Protazyme AK assay was started by transferring the tube to a prewarmed Eppendorf thermomixer set to 37°C. The tube was then incubated for 15 minutes on the thermomixer at 1100 rpm. The tube was transferred back to the ice-bath. 200µl cooled supernatant was transferred to a microtiter plate and OD405 was read in a VERSAmax microtiter reader from Molecular Devices. OD405 for the endopeptidase sample minus OD405 for a buffer blank was a measurement of the residual activity of the endopeptidase sample. If the OD405 measurement was above 1.5 OD405 units, the endopeptidase was diluted further, and the assay was repeated.

### Results:

The data correspond to the relative activities for each endopeptidase calculated as described above and listed in Table 10 taking the dilution factors into account.

**Table 10:**

| Enzyme | Example 1 stability | Example 3 stability |
|---|---|---|
| HZ-2 | 0.96 | 0.98 |
| HZ-3 | 1.05 | 1.09 |
| HZ-4 | 0.97 | 0.76 |
| HZ-6 | 0.99 | 0.75 |
| HZ-8 | 1.06 | 0.03 |
| HZ-9 | 0.98 | 0.84 |
| HZ-10 | 1.00 | 1.02 |
| HZ-11 | 0.93 | 0.19 |
| HZ-13 | 0.97 | 0.17 |
| HZ-15 | 0.96 | 1.09 |
| HZ-18 | 1.07 | 0.05 |
| HZ-19 | 1.01 | 0.87 |
| HZ-32 | 1.07 | 0.97 |
| HZ-35 | 1.04 | 1.03 |

It is seen that all enzymes were stable in conditions equivalent to Example 1. Therefore, the enzymes had been active during the whole incubation period.

It is seen that not all enzymes were stable in conditions equivalent to Example 3. This is because HZ-8, HZ-11, HZ-13 and HZ-18 had reduced activity after 4 hours at 30°C in incubation buffer B, suggesting that these endopeptidases were not 100% active during the whole incubation period. These data explain why HZ-13 underperformed as a glutamyl endopeptidase in Example 3.

### Example 5: Glutamyl endopeptidase has minor activity on the outer human skin layer protein

The present example describes an assay for evaluating how aggressive an endopeptidase is towards the outermost skin layer of the body that comprise the main biological barrier to enzyme or other pharmaceutical compound delivery to skin and hair follicles. The nine endopeptidases of SEQ ID NO: 1, 3, 6-8, 10 and 12-14 listed in Table 1 and Table 3 that showed targeted activity on the ORS tissue in Example 1, were analyzed in the present example to quantify the degree of stratum corneum protein hydrolysis for each enzyme.

Callous and thick skin consists of increased layers of dead keratinized cells in the outermost layer of the skin, stratum corneum. In terms of health, it is a protective response typically found on the palms and foot soles, or it can be causative of pathology in dermatological conditions. Granulated or powdered callous substrate was prepared using a metal scraper on the dry foot soles from a healthy human volunteer. The substrate was prepared by removing soluble protein using two washes with 0.01% Triton X-100. A 25 mg/mL suspension in 0.01% Triton X-100 was prepared and 200 µL suspension was pipetted into 1.5 mL Eppendorf tubes. The substrate was precipitated by a short centrifugation and the supernatants were discarded. The pellets were resuspended in 200 µl 0.01% Triton X-100 by agitation on an Eppendorf thermomixer (2000 rpm, 2 min) and the suspensions were centrifuged again, and the supernatants discarded. The second precipitates were resuspended in 180 µL HZ Buffer by Eppendorf mixing to prepare 190 µL suspensions, which were placed on ice. The endopeptidases were diluted in 0.01% Triton X-100 to 500 ppm and 10µL of each dilution was added to an Eppendorf tube with suspended skin substrate to give a final concentration of 25 ppm endopeptidase for the assay in duplicates. The Eppendorf tubes were incubated on a prewarmed Eppendorf thermomixer (30 min, 30°C, 2000 rpm) and after incubation the tubes were transferred back to the ice to cool for a few minutes. The tubes were centrifuged, and the supernatants were diluted 10x in 0.01% Triton X-100.

The degree of hydrolysis in the diluted supernatants for each endopeptidase was expressed as the solubilized protein concentration by measuring the absorbance at 562 nm using a PIERCE BCA Protein assay kit (23227 from Thermo Scientific). The results are shown in Table 11 below.

**Table 11:**

| Enzyme | Protein concentration (mg/mL) | | |
|---|---|---|---|
| | Sample 1 | Sample 2 | AVG |
| HZ-2 | 2.61 | 2.81 | 2.71 |
| HZ-4 | 0.82 | 0.76 | 0.79 |
| HZ-9 | 1.54 | 1.56 | 1.55 |
| HZ-10 | 9.88 | 9.48 | 9.68 |
| HZ-11 | 10.06 | 9.51 | 9.78 |
| HZ-13 | 1.10 | 1.06 | 1.08 |
| HZ-15 | 2.07 | 1.91 | 1.99 |
| HZ-19 | 8.54 | 8.77 | 8.66 |
| HZ-35 | 0.01 | 0.01 | 0.01 |

It can be observed in Table 11 that HZ-10, HZ-11 and HZ-19 hydrolyzed most of the outer skin layer. Too much unspecific hydrolysis is undesirable as it may breach the biological barrier to more than therapeutic enzymes. The glutamyl endopeptidases had 4-5x lower levels (HZ-2, HZ-15, HZ-13) and nearly no (HZ-35) hydrolytic activity on skin protein in the present conditions. It can be an advantage that enzymes included in the present invention have limited outer skin layer activity, as they are not only desired to disrupt the ORS to facilitate hair release, some selected activity is desired to facilitate enzyme delivery to hair follicles, occluded hair follicles with stratum corneum cell and keratin debris or delivery to hair follicle-linked lesions.

### Example 6: Glutamyl endopeptidase potently disrupts human ORS tissue of human beard hair

The present example illustrates how the unique selectivity of glutamyl endopeptidases translates into degradation of human ORS tissue and its keratin components responsible for causing hair follicle-linked diseases. The two endopeptidases of SEQ ID NO: 1 (HZ-2) and SEQ ID NO: 7 (HZ-10) listed in Table 1 that showed activity in Example 1 and Example 3, and were stable in Example 4, were selected for analysis by microscopy in the present example, to evaluate their histological activity after enzyme incubation.

Freshly plucked beard hairs were collected in samples of 10 and fixed on glass slides (Figure 3). A total of 6 samples were prepared. One sample was left untreated and directly stained and imaged (8). The other hair samples were incubated in the following solutions. One sample was a negative control (200 µl 50 mM Tris pH 8, 2mM CaCl₂ buffer (9)), two samples were incubated with HZ-2 (200 µl 1 ppm HZ-2 (10) or 200 µl 30 ppm HZ-2 (11) in the same buffer), and two samples were treated with HZ-10 (200 µl 1 ppm HZ-10 (12) or 200 µl 30 ppm HZ-10 (13) in the same buffer). The glass slides were covered to avoid evaporation and incubated at 30°C for 3 hours. The hairs were stained with the red 1% DMACA in 0.5M HCI IRS stain and the purple/blue ORS stain comprising a 1:1 mixture of 2.5mg/ml Toluidine blue in Mcllvane buffer pH=3.6 and 0.1% Rhodamine B in Walpoles buffer pH=4.4 and immediately imaged using a light microscope and 40X magnification. It can be observed from the 2-3 representative hairs in in each sample of Figure 3 that the ORS of the negative control (9) was unchanged relative to the untreated control (8). For the 30 ppm HZ-10 incubation (13) only the fully keratinized hair shaft was left after incubation and for the 1 ppm HZ-10 incubation (12) the less resistant, but still keratinized IRS could be seen. For the 30 ppm HZ-2 incubation (11) the inner part of the ORS was seen after incubation and for the 1 ppm HZ-2 incubation (10) it is evident that the ORS tissue was only hydrolyzed enough to swell to double the size of the negative control. The data suggests that, compared to HZ-10, HZ-2 potently disrupts the ORS in a less aggressive way from the perspective of cell and tissue disruption, despite its narrower specificity. Selective tissue disruption is preferred for the present invention as it limits unwanted side reactions to ensure safer treatments.

### Example 7: Glutamyl endopeptidase potently hydrolyze type I and II keratins in human ORS tissue

The present example documents the glutamyl endopeptidase protein substrates of the human ORS tissue. The endopeptidases of SEQ ID NO: 1 (HZ-2) listed in Table 1 that gave a positive response in Example 1 and Example 3 and that was stable in Example 4 and showed significant activity in Example 5 and Example 6, was analyzed using SDS-PAGE to investigate its ability to specifically hydrolyze ORS tissue protein.

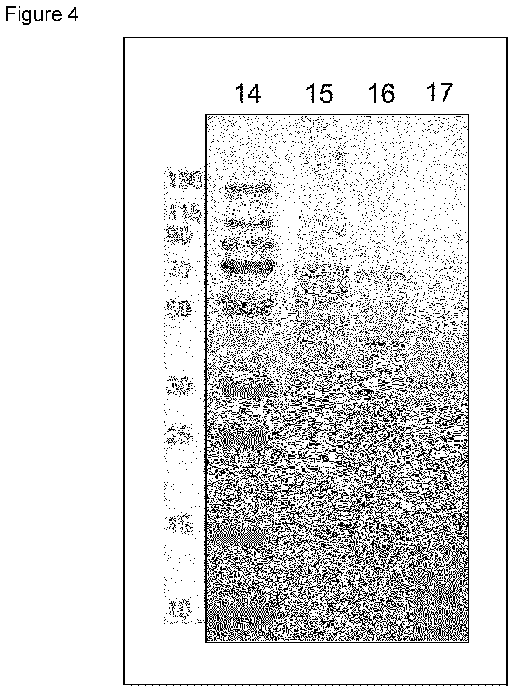

Newly plucked beard hairs were collected in samples of 5 in Eppendorf tubes. A total of 3 samples were prepared (Figure 4). The samples were incubated in buffer for 30 min at 30°C. One sample was the negative control (50 mM Tris/HCl, pH 8, 0.1% Triton X-100 and 2 mM CaCl₂ buffer), one sample was incubated with HZ-2 (200 µL 1 ppm in the same buffer) and one sample was incubated with HZ-2 (200 µL 5 ppm in the same buffer). After the incubation, the supernatant was carefully removed, and the remaining tissue was lysed in 8M urea, 2% SDS, 100 mM DTT and 100 mM Tris/HCl, pH 8 for 30 min at 30°C. Due to the less dissolvable character of the IRS and hair shaft, the far majority of protein extracted is derived from ORS, which was confirmed by observing the lack of ORS under the microscope before and after lysis (data not shown). Proteins were concentrated using a 10 kDa molecular cut-off spin filter and separated using SDS-PAGE stained with Coomassie Blue to visualize the proteome. A MES buffer and a 10% Bolt Bis-Tris Plus gel was used. The results are shown in Figure 4. A PageRuler Plus Prestained Protein ladder (14) (ThermoFischer Scientific) was used to identify the approximate absolute sizes corresponding to the bands in the gel. The negative control (15), 1 ppm HZ-2 (16) and 5 ppm HZ-2 (17) were loaded equally. It can be observed that there were two intensely staining groups of bands that a person skilled in the art will conclude corresponds to mainly type I (50-60 kDa) and type II (60-70 kDa) keratins of the ORS. In the present conditions just 1 ppm HZ-2 degraded the type I keratins, whereas 5 ppm HZ-2 fully degraded type I and type II during the experiment, both leaving multiple bands intact. The results indicate that HZ-2 is a potent and selective ORS keratinase. Since type I and type II keratins of the ORS are required for hair anchoring, are the effector molecules of follicular hyperkeratosis and occlusion, and supports the function and integrity of the hair follicle, their selective hydrolysis by HZ-2 explains the observed hair release in Example 1 and 3 and how compositions of glutamyl endopeptidases such as HZ-2 can be used to treat hair follicle-linked conditions and follicular dermatoses.

### Example 8: Glutamyl endopeptidase selectively disrupts ORS tissue in healthy human skin

This example illustrates the selective disruption of ORS tissue by glutamyl endopeptidases in human skin. The two endopeptidases of SEQ ID NO: 1 (HZ-2) and SEQ ID NO: 7 (HZ-10) listed in Table 1 that was able to release hair in Example 1 and Example 3 and that were stable in Example 4 and showed significant activity in Example 5 and 6, were analyzed by histological evaluation after enzyme incubation.

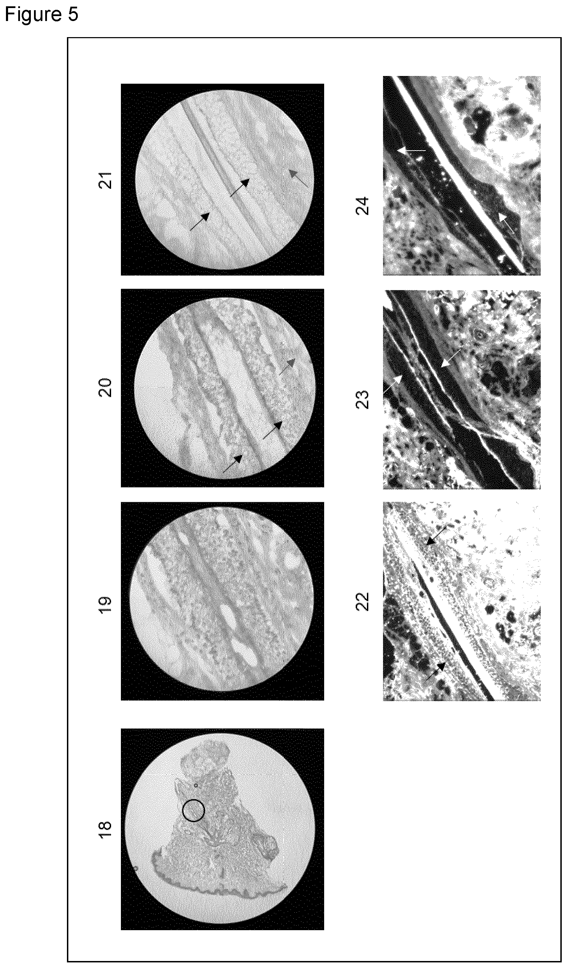

A 6mm punch axillary skin biopsy from a healthy female volunteer was cryosectioned into 10 µm slices using a microtome. The skin slices were placed on glass slides for microscopy. Three representative neighboring skin slices cut transversely through a hair follicle were prepared, and an overview is showed at 40x magnification in Figure 5 (18). The tissue slices were incubated in a droplet of buffer solution on the slides and covered to prevent evaporation for 30 min at 30°C and visualized at 400x magnification using light microscopy from the circled area in (18) around the hair shaft and ORS. One slice was a negative control (50 mM Tris/HCl pH 8 and 2mM CaCl₂ buffer (19)), next slice was incubated with HZ-2 (100 ppm HZ-2 in the same buffer (20)), and the next slice was incubated with HZ-10 (100 ppm HZ-10 in the same buffer (21)). After incubation the slices were stained with hematoxylin and eosin (18-21) or with 2.5mg/ml Toluidine blue in Mcllvane buffer pH=3.6 and 0.1% Rhodamine B in Walpoles buffer pH=4.4 for 10 min, followed by 1% Rhodamine B in Walpoles buffer pH=4.4 for 1 min (22-24). It can be observed from the data presented in Figure 5 that HZ-2 (20) selectively disrupted the ORS tissue into cell clumps (black arrows), suggesting a mainly intercellular ORS effect compared to the negative control while leaving the dermis intact and comparable to negative control (gray arrow). However, HZ-10 (21) destroyed the ORS cell content, while apparently keeping the ORS tissue intact, suggesting a mainly intracellular ORS effect (black arrows), while visibly eliminating the nuclei present in the collagen of the dermis (gray arrow). Using fluorescence microscopy at 200x magnification exciting the Rhodamine B stain with green light provides improved contrast and revealed that HZ-2 (23, ORS in white arrows) in contrast to the negative control (22, ORS in black arrows) and HZ-10 (24, ORS in white arrows) under the present conditions also did not significantly disrupt non ORS tissue that had undergone epidermal keratinization (more white tissue in (22, 23) than (24)) such as the IRS (white arrows in (23, 24)) and epidermis (not shown). This suggests that HZ-2 had significantly less off-target activity than HZ-10, while effectively disrupting the ORS tissue. The effect seen by HZ-2 is most desired for effective and safe treatment of hair follicle-linked conditions and follicular dermatoses as described for the compositions and use in the present invention.

### Example 9: Glutamyl endopeptidase selectively disrupts ORS tissue in lesional Hidradenitis suppurativa skin

This example illustrates the selective disruption of ORS tissue by glutamyl endopeptidases in Hidradenitis suppurativa patient skin. The two endopeptidases of SEQ ID NO: 1 (HZ-2) and SEQ ID NO: 7 (HZ-10) listed in Table 1 that gave positive responses in Example 1 and Example 3 and that were stable in Example 4 and showed significant activity in Example 5-6 and Example 8, were used for treating the patient tissue which subsequently was analyzed by histological evaluation after enzyme incubation.

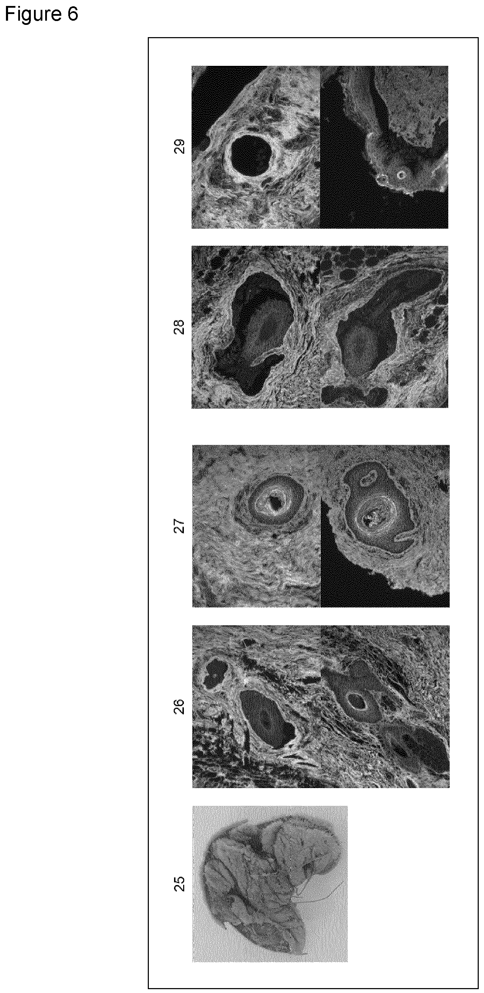

A 10x10x8 mm axillary lesional skin resection biopsy (Figure 6, (25)) from a female volunteer suffering from moderate Hidradenitis suppurativa with clear inflammatory characteristic was incubated fully suspended in negative control (125 mM Tris/HCl pH 8, 0.25% Triton X-100 and 5 mM CaCl₂ buffer or HZ-2 (500 ppm HZ-2 in the same buffer) for 3 hours at 30°C. Samples were fixed in 10% formalin for a week before being dried using vacuum and embedded in paraffin. Samples were sectioned into 4 µm slices after locating skin appendages onto glass slides using a microtome, samples were de-paraffinized by melting for 1 hour at 60°C, washed in Tissue Clear, 99% ethanol, 70% ethanol and deionized water. Samples were stained after re-fixing the tissue in Boin's fixative overnight, washed thoroughly with deionized water, stained with fresh Weigert's iron hematoxylin for 10 min, washed with deionized water, counterstained with picric acid 10% Sirius Red for 15 min and dehydrated 2x in 99% ethanol.

It can be observed from the 200x zoom fluorescence microscopy images after exciting Sirius Red presented in Figure 6, two different representative slices per treatment condition, that the negative control prelesional hair follicles (26) with evidence of cornification, occluded follicular cysts (27), whereas HZ-2 selectively disrupted the ORS tissue in two pre-lesional hair follicles (28) leaving ORS cells separated around the hair shaft, and leaving the surrounding dermis intact. HZ-2 also appeared to degrade early stage follicular cysts (29) by leaving them empty. In conclusion, like the ORS from healthy patients in Example 8, HZ-2 is active on lesional and likely pre-cystic ORS tissue from Hidradenitis suppurativa patients and is thus also likely to have the same effect on tissue from patients suffering from the multiple hair follicle-linked conditions and follicular dermatoses targeted for treatment by the compositions and uses of the present invention.

### Example 10: Glutamyl endopeptidase facilitates topical follicular delivery of protein

This example shows that compositions with HZ-2 facilitates follicular delivery of high molecular weight compounds such as proteins (here the enzyme itself) as shown by increased penetration of reflective nanoparticles inside the hair follicles of hairy pig skin. The two endopeptidases of SEQ ID NO: 1 (HZ-2) and 7 (HZ-10) listed in Table 1 that gave a positive response in Example 1 and Example 3 and that were stable in Example 4 and showed significant activity in Examples 5-6 and Examples 8-9, and the tissue was analyzed by reflectance confocal laser microscopy.

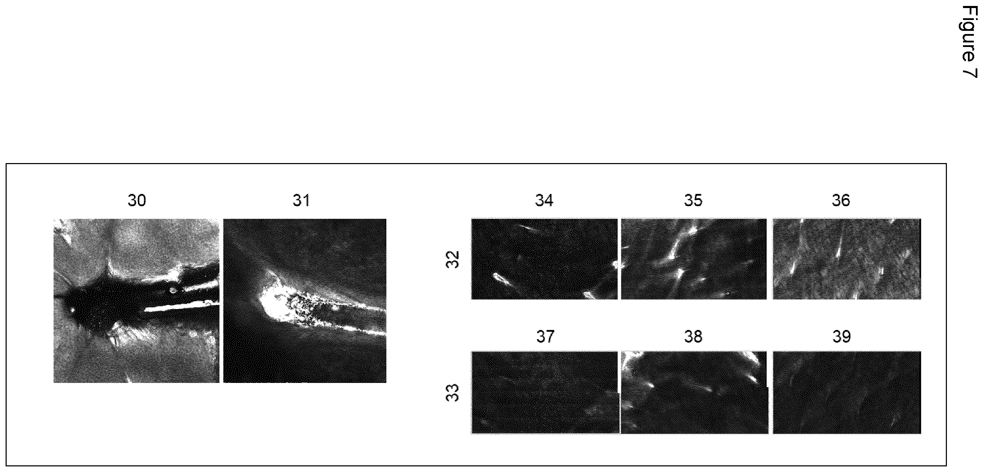

In Figure 7, a buffer comprising 25 mM Tris/HCl, pH 8, 150 mM NaCl, 1% Span 20 surfactant, 2mM potassium sorbate, 5 mM CaCl₂, was used as negative control. The buffer was very similar to the compositions used in the topical experiment in Example 3, only lacking DTT. Negative control, HZ-2 (500 ppm HZ-2 in the same buffer) and HZ-10 (500 ppm HZ-10 in the same buffer) were added strictly topically to the dorsal side of pig ears as shown in Figure 2 (6) and Example 3, however without prior microneedle perforation. The skin sections were incubated for 20 hours at 4°C followed by 5 hours at 30°C within glued on PMMA squares. After discarding the buffer and removing the PMMA squares the resulting enzyme-treated pig skin samples were kept on ice for up to 2 hours until analyses started.

Hairs were trimmed to 3 mm length and 120 nm silica core gold nanoparticles (GNP) with a peak absorption at 800 nm (CE 612960, SEB-250, Sebacia Inc.) were massaged into the skin using a gloved finger for 2 min. GNP penetration was used as reporter for skin and follicular ORS enzyme activity by analyses using a 830 nm reflectance confocal laser microscope *en face* (Figure 7). GNPs enhance the contrast of the microscopy images by having increased reflectance around the hairs in the images as they penetrate the follicle. Scans were performed at 0 µm without (30) and after adding GNPs (31). During the experiment, reflectance at 188 µm (32) and 300 µm (33) below the skin surface were measured to evaluate GNP penetration depth for each sample.

It was found that the GNPs only penetrated to a depth of 188 µm (34) but not 300 µm for the negative control (37). Remarkably, HZ-2 allowed the GNPs to penetrate to a depth of 300 µm (35, 38), which is equivalent to a large fraction of the hair follicle infundibulum and the site of hair follicle-linked action that enzymes need to be delivered to treat follicular dermatoses like Hidradenitis suppurativa. Yet, the more unspecific HZ-10 failed to pass beyond 188 µm (36) leaving no trace of GNPs at 300 µm depth (39). 300 µm was the technical limit of the confocal laser microscope suggesting that HZ-2 penetrates even deeper. The example further illustrates the ability of HZ-2 to increase the delivery of nanoparticles to hair follicles, which is a desired way to formulate enzyme-based and other drugs, to treat diseases by follicular or in some cases transfollicular delivery. The present example further illustrates the possibility of using HZ-2 to enhance the permeability of other small and large compounds to hair follicles, including proteins such as the HZ-2 enzyme itself or any other protein stable in the presence of glutamate-specific HZ-2 or any pharmaceutical that would benefit industrially from being delivered topically.

## Claims

1. A composition, which is suitable for topical application to human skin, comprising one or more enzymes, where the composition is:
a. a pharmaceutical composition, comprising at least one pharmaceutically acceptable ingredient in addition to the one or more enzymes,
or
b. a cosmetic composition, comprising at least one cosmetically acceptable ingredient in addition to the one or more enzymes,
wherein the one or more enzymes are glutamyl endopeptidases with a glutamyl endopeptidase ratio (GR) > 5, where GR is calculated as the enzyme activity on substrate Suc-AAPE-pNA divided by the highest of activities on substrates Suc-AAPA-pNA, Suc-AAPD-pNA, Suc-AAPF-pNA, Suc-AAPI-pNA, Suc-AAPK-pNA, Suc-AAPL-pNA, Suc-AAPM-pNA, Suc-AAPR-pNA, and Suc-AAPV-pNA.

2. The composition of claim 1, wherein the glutamyl endopeptidases are selected among glutamyl endopeptidases having at least 80% sequence identity with SEQ ID NO: 12 or SEQ ID NO: 14.

3. The composition of claim 2, wherein the sequence identity is at least 90%, such as at least 95% sequence identity, at least 96% sequence identity, at least 97% sequence identity, at least 98% sequence identity, and at least 99% sequence identity.

4. The composition of claim 2, wherein the glutamyl endopeptidases are selected among the glutamyl endopeptidases having an amino acid sequence comprising or consisting of SEQ ID NO: 2 or SEQ ID NO: 14, or a variant thereof comprising 1, 2, 3, 4, 5, 6, 7, 8, 9, or 10 substitutions in comparison with SEQ ID NO: 12 or 14.

5. The composition according to any of the preceding claims, wherein the glutamyl endopeptidases are present in amounts in the range of 1 ng to 100 mg enzyme protein per g of the composition.

6. The composition according to any of the preceding claims, wherein the composition is formulated as an emulsion, a suspension, or a solution.

7. A medical device comprising a pharmaceutical composition according to any of the preceding claims, wherein the medical device is a patch or hollow needle comprising the composition.

8. The pharmaceutical composition according to any one of claims 1-6 for use in treatment of follicular dermatoses or hair follicle-linked conditions.

9. The pharmaceutical composition for the use according to claim 8, wherein the hair follicle-linked condition and follicular dermatoses is selected among: Hirsutism, Hypertrichosis or Pseudofolliculitis barbae; or follicular dermatoses that are induced by follicular hyperkeratosis or follicular occlusion that leads to development of Acne vulgaris, Keratosis pilaris, Dowling-Degos disease, Hailey-Hailey disease, Mammillary fistula or follicular occlusion tetrad syndromes including Acne conglobata, Pilonidal sinus disease, Dissecting cellulitis of the scalp and Hidradenitis suppurativa; or inflammatory hair follicle-linked conditions including Follicular psoriasis, Pityriasis Rubra Pilaris, Discoid Lupus, Lichen Planopilaris, Hypertrophic Lichen Planopilaris, Lichen Planus Follicularis Tumidus, Lichen Schlerosus, Lichen Spinulosus, Wong-type dermatomyositis, infectious hair follicle-linked conditions including Post-Kala Azar dermal Leishmaniasis, Type I Reactions in borderline tuberloid leprosy, Pityriasis Folliculorum; and other hair follicle-linked diseases including Nevus Comedonicus, Folliculotropic Mycosis Fungoides and Phyrnoderma.

10. Use of a cosmetic composition according to any one of claims 1-6 for removal of undesired hair.

11. The pharmaceutical composition according to any one of claims 1-6 for use as a medicament.
